# EUROPEAN PATENT APPLICATION

(11) **EP 4 368 729 A1**
(43) Date of publication of application: **15.05.2024**
(21) Application number: 22206405.7
(22) Date of filing: 09.11.2022
(51) Int. Cl.: C12Q 1/6883

(54) **MOLECULES TARGETING RIBOSOMAL PROTEIN RPL40 FOR USE IN COSMETICS AND THE TREATMENT OF DISEASES OF THE SKIN**

(71) Applicant: Merck Patent GmbH, 64293 Darmstadt (DE)
(72) Inventor: Von Hagen, Joerg, 64293 Darmstadt (DE); Breitenbach Koller, Hannelore, 5020 Salzburg (AT)
(74) Representative: Merck Patent Association

(57) **Abstract**

The present invention relates to a method for identifying a pharmaceutically or cosmetically active compound that modulates the rpL40-dependent translation of at least one mRNA encoding for tropoelastin in a mammalian cell. Furthermore, a respective screening system, methods of treating or preventing a disease or condition, and compounds as identified that modulate the rpL40-dependent translation as well synergistic combinations thereof are provided. In a preferred aspect, the cosmetically active compound is used in anti-aging products and strategies.

## Description

The present invention relates to a method for identifying a pharmaceutically or cosmetically active compound that modulates the rpL40-dependent translation of at least one mRNA encoding for tropoelastin in a mammalian cell. Furthermore, a respective screening system, methods of treating or preventing a disease or condition, and compounds as identified that modulate the rpL40-dependent translation as well synergistic combinations thereof are provided. In a preferred aspect, the cosmetically active compound is used in anti-aging products and strategies.

### Background of the invention

Elastin, a skin protein complex built from tropoelastin monomers, is the major component of elastic fibers, which provide elasticity to the skin to stretch and recoil. In combination with subcutaneous fat and other cutaneous extracellular matrix proteins, such as proteoglycans, and glycosaminoglycans, elastin is required for maintaining healthy skin structure and function.

Elastic fibers are assembled through a process known as elastogenesis. Elastogenesis takes place in in elastogenic cell types, such as fibroblasts, and is dependent on the expression, assembly, and crosslinking of its dominant component, tropoelastin (Wise SG, Weiss AS. Tropoelastin. Int J Biochem Cell Biol. 2009 Mar;41(3):494-7. doi: 10.1016/j.biocel.2008.03.017. Epub 2008 Apr 1. PMID: 18468477, Vindin H, Mithieux SM, Weiss AS. Elastin architecture. Matrix Biol. 2019 Nov;84:4-16. doi: 10.1016/j.matbio.2019.07.005. Epub 2019 Jul 10. PMID: 31301399). Elastogenesis starts with soluble tropoelastin monomers, which are secreted by and bound to fibroblasts receptors. Tropoelastin then aggregates into microscopic globules, a process termed coacervation. During this process, tropoelastin molecules are oxidized by different members of the lysyl-oxidase family. This induces crosslinking to form the elastin polymer. Elastin then associates with fibrillin-rich microfibrils to form a nascent elastic fiber which is released from the cell surface into the extracellular matrix (Kielty CM, Wess TJ, Haston L, Ashworth JL, Sherratt MJ, Shuttleworth CA. Fibrillin-rich microfibrils: elastic biopolymers of the extracellular matrix. J Muscle Res Cell Motil. 2002;23(5-6):581-96. PMID: 12785107).

Elastin is a long-lived protein with little turnover, and in skin overall half-life of elastin is similar to human life span (Uitto J, Li Q, Urban Z. The complexity of elastic fibre biogenesis in the skin--a perspective to the clinical heterogeneity of cutis laxa. Exp Dermatol. 2013 Feb;22(2):88-92. doi: 10.1111/exd.12025. Epub 2012 Oct 23. PMID: 23088642; PMCID: PMC3556375). However, moderate neo-synthesis of the monomeric precursor tropoelastin occurs in adult and aged skin cells, and early studies showed that steady state, basal levels of elastin production and breakdown are observed in adult tissues as well as in cells isolated from adult tissues (Starcher BC. Elastin and the lung. Thorax. 1986 Aug;41(8):577-85. doi: 10.1136/thx.41.8.577. PMID: 3538485; PMCID: PMC460400, Parks WC, Secrist H, Wu LC, Mecham RP. Developmental regulation of tropoelastin isoforms. J Biol Chem. 1988 Mar 25;263(9):4416-23. PMID: 3346253).

The degradation of elastic fibers starts with advancing age and exposure to environmental injuries. This degradation of the skin's structural integrity, combined with subcutaneous fat loss, results in looser, sagging skin, causing undesirable changes in appearance.

Damaging changes occur in chronically sun-exposed skin, which shows deregulation both in amount and arrangement of cutaneous elastic fibers and loss of fine elastic fibers in the superficial dermis, required for flexible connection to the epidermis. However, the most devasting effect on skin elasticity results from the replacement of the normal collagen-rich superficial dermis with abnormal clumps of solar elastosis material formed upon prolonged exposure to UV light. In an analogous fashion, disruption of elastic fiber networks after injury and during wound healing leads to undesirable characteristics in structure, and appearance of scars and stretch marks. During wound closure, abnormally arranged large bundles of collagen are deposited to promote wound closure. While injury also induces re-initiation of tropoelastin expression, elastic fibers are the last extracellular matrix fibers that arrive at the scene during the remodeling phase of scar tissue and are not produced at levels required to establish a true reformation of the elastic fiber network with elasticity comparable to the original tissue.

Nguyen, H.L., et al. (in: Erythromycin leads to differential protein expression through differences in electrostatic and dispersion interactions with nascent proteins. Sci Rep 8, 6460 (2018). https://doi.org/10.1038/s41598-018-24344-9) examine interactions of the macrolide erythromycin with the ribosome.

US 7,927,791 relates to a method for screening and identifying compounds that modulate premature translation termination and/or nonsense-mediated messenger ribonucleic acid ("mRNA") by interacting with a preselected target ribonucleic acid ("RNA"). In particular, the document relates to identifying compounds that bind to regions of the 28S ribosomal RNA ("rRNA") and analogs thereof.

WO 2012/142542 relates to methods to identify molecules that binds in the neomycin binding pocket of a bacterial ribosome using structures of an intact bacterial ribosome that reveal how the ribosome binds tRNA in two functionally distinct states.

Lee A.S.-Y., Kerr R.B.K., and Whelan S.P.J. (in: A ribosome-specialized translation initiation pathway is required for cap-dependent translation of vesicular stomatitis virus mRNAs. Proc. Natl. Acad. Sci. U. S. A. 2013;110:324-329) identify the large ribosomal subunit protein rpL40 as requisite for VSV cap-dependent translation but not bulk cellular or internal ribosome entry site-driven translation.

US9878056B2 relates to cosmetic mRNAs encoding elastin, and methods of using such mRNAs.

US9458197B2 discloses a method of treating patient's facial lines and wrinkles, the method comprising administering to a site on the patient in need thereof an effective amount of a composition comprising one of three elastin peptides as disclosed, and a combination thereof.

In view of the above, there is thus a great medical and cosmetic and aesthetic need for interventions to replenish elastin and elastic fibers to improve the appearance, texture, resiliency, and wound-healing capabilities in damaged or aging skin. While there are some interventions available that improve the levels of collagen and hyaluronic acid in the skin, there is no established treatment yet that increases production of elastin in skin cells and the skin. It is therefore an object of the present invention to provide new therapeutic and cosmetic approaches, methods and means. Other objects and advantages of the present invention will become apparent to the person of skill when studying the following more detailed description of the present invention, including the Figures and examples.

The present invention avoids the caveats of commonly used routes used for development of small molecules in systemic therapy. A cellular screen to identify a target ribosomal protein was used that can be manipulated by a drug ligand to customize an increase in protein expression of tropoelastin isoforms with minimal interference to bulk protein expression.

In a first aspect of the present invention, the above object is solved by a method for identifying a cosmetically and/or pharmaceutically active compound that modulates the rpL40-dependent translation of at least one mRNA encoding for a tropoelastin in a mammalian cell, comprising
a) contacting rpL40 or a functional fragment thereof with at least one candidate compound in the presence of said at least one mRNA to be translated, and
b) detecting the modulation of the translation of said at least one mRNA encoding for a tropoelastin compared to the translation in the absence of said at least one candidate compound, wherein a modulation of the translation of said at least one mRNA is indicative for a cosmetically and/or pharmaceutically active compound.

Preferably, the method furthermore comprises detecting a binding of said at least one candidate compound to a fragment of rpL40, wherein said fragment comprises from about 30 to 90, preferably 60 to 80 of the C-terminal amino acids of the mammalian rpL40 protein, preferably according to SEQ ID NO: 2, and the detecting said binding is performed as a pre-screening before contacting said at least one candidate compound with said rpL40.

Bauer et al. (in: Specialized yeast ribosomes: a customized tool for selective mRNA translation. PLoS One. 2013; 8(7):e67609. doi:10.1371/journal.pone.0067609) describe diploid yeast strains, each deficient in one or other copy of the set of ribosomal protein (RP) genes, to generate eukaryotic cells carrying distinct populations of altered 'specialized' ribosomes. Using the strains, a screen identified specialized ribosomes with reduced levels of RP L35B as showing enhanced synthesis of full-length LAMB3 in cells expressing a LAMB3-PTC mutant. It was speculated that the rational modification of a eukaryotic ribosome could customize increased translation of a specific, disease-associated mRNA and may represent a novel therapeutic strategy for the future.

Tropoelastin is the monomer of elastin, the main component of the elastic fibers that provide the skin with elasticity and the ability to stretch and recoil. Physiological levels of elastic fiber production, the correct organization, and integration with other cutaneous extracellular matrix proteins, proteoglycans, and glycosaminoglycans are integral to maintaining healthy skin structure, function, and youthful appearance (Baumann L, Bernstein EF, Weiss AS, Bates D, Humphrey S, Silberberg M, Daniels R. Clinical Relevance of Elastin in the Structure and Function of Skin. Aesthet Surg J Open Forum. 2021 May 14;3(3):ojab019. doi: 10.1093/asjof/ojab019. PMID: 34195612; PMCID: PMC8239663). Although elastin has very low turnover, its production decreases after individuals reach maturity and are at low levels during later age. With advancing age (intrinsic factors) and exposure to environmental insults (extrinsic factors) elastic fibers degrade. In combination with subcutaneous fat loss, this results in looser, sagging skin, which loses elasticity. This is aggravated in chronically sun-exposed skin, which increases tropoelastin expression in the epidermis and dermis to unphysiological levels. However, since the enzymatic activities organizing the formation of higher-ordered elastin are absent, there is no proper formation of cutaneous elastic fibers. The thus accumulating, abnormal molecular debris of solar elastosis replaces the normal collagen-rich superficial dermis and displaces the fine elastic fibers in the superficial dermis connecting to the epidermis. Such a disruption of elastic fiber networks also leads to undesirable characteristics in wound healing, which is accompanied by worsening of skin structure and appearance of scars and stretch marks. A multitude of interventions have thought to replenish elastin and elastic fibers to improve the skin's appearance, texture, resiliency and wound-healing capabilities. However, at present there is no systemic or other therapy that is capable to replenish endogenous tropoelastin production and proper formation of elastic fibers at physiological levels in order to, for example, sustain the elastin fiber network required for youthful appearance of human skin and/or to ameliorate scars during wound healing.

In a second aspect of the present invention, the above object is solved by a providing a screening system for identifying a cosmetically and/or pharmaceutically active compound that modulates the rpL40-dependent translation of at least one mRNA encoding for a tropoelastin in a mammalian cell, comprising an eukaryotic cell recombinantly expressing a mammalian rpL40 or a fragment of a mammalian rpL40, wherein said fragment comprises from about 30 to 90, preferably 60 to 80 of the C-terminal amino acids of the mammalian rpL40 protein, preferably according to SEQ ID NO: 2, an expression construct for recombinantly expressing the at least one mRNA to be tested, and optionally, one or more candidate compounds to be tested.

In a third aspect of the present invention, the above object is solved by providing a compound that modulates the rpL40-dependent translation of at least one mRNA encoding for a tropoelastin in a mammalian cell for use in the prevention or treatment of diseases, in particular a disease or condition caused by a lack and/or insufficient synthesis of functional elastin, wherein said disease or condition is preferably selected from pulmonary hypertension, cigarette smoke induced emphysema, undesirable would healing, and severe chronic obstructive pulmonary disease (COPD), and wherein said compound was identified with a method according to the present invention. In particular, the compound may be selected from the group of compounds selected from the following formulae: 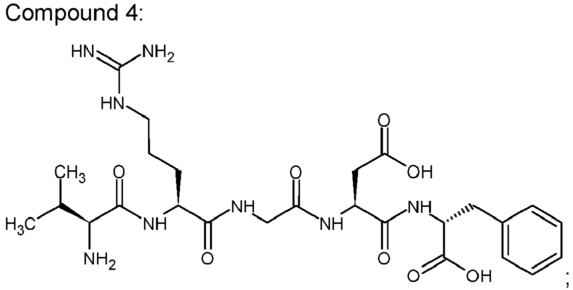 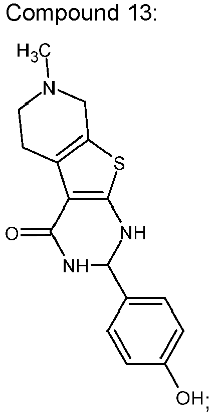 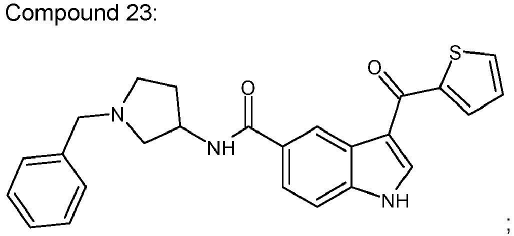 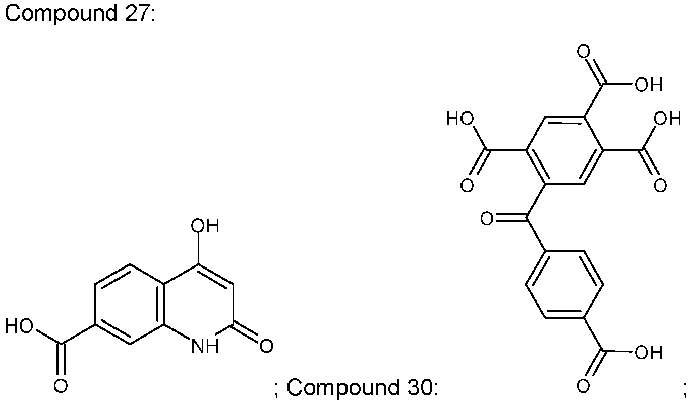 and 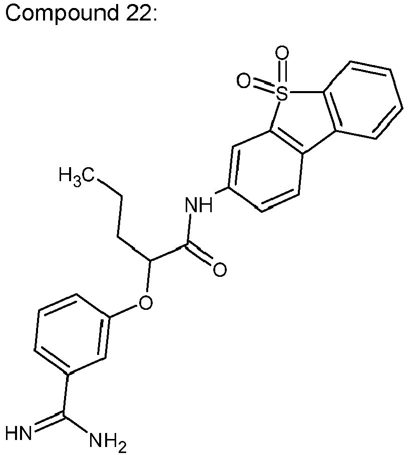 and derivatives thereof, and cosmetically and/or pharmaceutically acceptable salts thereof.

Further preferred is a subgroup of chemically related compounds 13, 23, 27, and 4 and derivatives thereof, and cosmetically and/or pharmaceutically acceptable salts thereof, that exhibit a tautomeric group located within about 3-4Å distance to an acidic group in the same molecular plane.

In a fourth aspect of the present invention, the above object is solved by a cosmetic and/or pharmaceutical composition comprising at least one compound modulating the rpL40-dependent translation of at least one mRNA encoding for a tropoelastin in a mammalian cell according to the present invention, together with at least one cosmetically and/or pharmaceutically acceptable carrier, preferably for systemic or topical administration.

In a fifth aspect of the present invention, the above object is solved by a method, in particular a cosmetic or non-medical method, for modulating the rpL40-dependent translation of at least one mRNA encoding for a tropoelastin in a mammalian cell or tissue, comprising contacting said cell or tissue with an effective amount of a compound that was identified with a method according to the present invention, or the cosmetic and/or pharmaceutical composition according to the present invention. Preferred is a method, wherein said modulating the rpL40-dependent translation of at least one mRNA encoding for a tropoelastin is for preventing or treating a condition selected from photo ageing, skin rejuvenation, and increasing functional elastin synthesis.

In a sixth aspect of the present invention, the above object is solved by a method of preventing, treating or ameliorating a disease or condition caused by a lack and/or insufficient synthesis of functional elastin, wherein said disease or condition is preferably selected from pulmonary hypertension, cigarette smoke induced emphysema, and severe chronic obstructive pulmonary disease (COPD), comprising administering an effective amount of a compound that was identified with a method according to the present invention, or of the pharmaceutical composition according to the present invention to a patient or subject in need of said treatment or prevention.

The present invention is based on the surprising finding that human ribosomal protein rpL40 can be used as a target for a tailor-made modulation of the translation of certain mRNAs into proteins, here tropoelastin encoding mRNAs. At present, there is no technology that by systemic modification of a ribosomal protein customizes the ribosome for increase or decrease in protein production of a given protein. Boosting or reduction of protein species is desirable medical applications, in biotechnological applications and in cosmetic and anti-aging interventions.

Ultimately, the present invention is in the field of specialized ribosomes and targeting ribosomal proteins (RP) offers new routes for the treatment of severe inherited diseases such as EB (see also: Dalla Venezia N., et al. Emerging Role of Eukaryote Ribosomes in Translational Control. Int J Mol Sci. 2019;20(5):1226. Published 2019 Mar 11. doi:10.3390/ijms20051226; Ferretti MB, Karbstein K. Does functional specialization of ribosomes really exist?. RNA. 2019;25(5):521-538. doi:10.1261/rna.069823.118). Recent results have underscored the importance of translational control in regulation of gene expression, augmenting the traditional role of transcription. Mis-regulation of translation is a leading cause of many diseases. Viruses use a variety of mechanisms to co-opt the translational machinery to facilitate their replication, including manipulation of translation initiation factors, and specific RNA structures to guide translation within their genomes. Translational control has been implicated in human cancer, with changes in protein synthesis caused by up-regulation or changed functions of initiation factors. Finally, many genetic diseases disrupt translation through premature termination codons (Chen J, et al. The molecular choreography of protein synthesis: translational control, regulation, and pathways. Q Rev Biophys. 2016;49:e11. doi:10.1017/S0033583516000056). This invention provides progress towards this concept in order to harness the potential of targeting translation therapeutically.

EP2251437 (incorporated herein by reference) discloses a two-step specialized ribosome screen (2SSRC) which is able to screen for ribosomal protein targets specifically regulating protein synthesis of a protein of interest. These screens provide a direct readout of protein synthesis. The assay used in the screen can be employed for all follow up steps including pre-clinical studies, or testing a small molecule binder which targets the ribosomal protein for customizing protein expression. In yeast and human cells, subpopulations of cytoplasmic ribosomes can be generated, by providing altered functional availability of individual ribosomal proteins. Such heterologous or specialized ribosomes are tailored to increase or decrease protein expression of selected mRNAs, while leaving bulk protein expression unaltered.

In a first aspect of the present invention, a method for identifying a cosmetically and/or pharmaceutically active compound that modulates the rpL40-dependent translation of at least one mRNA encoding for a tropoelastin in a mammalian cell ("modulator") is provided. The method comprises the steps of contacting rpL40 or a functional fragment thereof with at least one candidate compound in the presence of said at least one mRNA to be translated and detecting the modulation of the translation of said at least one mRNA compared to the translation in the absence of said at least one candidate compound. A modulation of the translation of said at least one mRNA as detected is then indicative for said cosmetically and/or pharmaceutically active compound.

The term "contacting" in the present invention means any interaction between the potential modulator with the ribosomal protein or fragment thereof as described herein and/or a recombinant cell expressing said ribosomal protein or fragment thereof, whereby any of the two components can be independently of each other in a liquid phase, for example in solution, or in suspension or can be bound to a solid phase, for example, in the form of an essentially planar surface or in the form of particles, pearls or the like. In a preferred embodiment, a multitude of different potentially binding candidate compound are immobilized on a solid surface like, for example, on a compound library chip, and the ribosomal protein or fragment thereof as described herein is subsequently contacted with such a chip.

The method according to the present invention seeks to a pharmaceutically active compound that modulates the rpL40)-dependent translation of at least one mRNA encoding for a tropoelastin in a mammalian cell ("modulator"), preferably in a human cell, such as a keratinocyte. Conveniently, the format of the method can be quite flexible, the method requires a suitable combination of the three components rpL40 (either isolated or in combination with other ribosomal components) or a functional fragment thereof, the at least one tropoelastin mRNA to be translated, and the at least one cosmetically and/or pharmaceutically active candidate compound, i.e. the substance that shall be screened/identified for the activity to modulate the rpL40-dependent translation of at least one mRNA in a mammalian cell. This combination can be provided as a cellular system (i.e. functioning in a cell, like in a yeast or mammalian cell culture, such as a keratinocyte), and the components can be provided recombinantly in part or fully, or the system can be in vitro, for example as an in vitro translation system that can be readily adjusted for the purposes of the present invention, if required. Examples are Hela cell in vitro translation assays and human keratinocyte model cells.

Preferred is the method according to the present invention, which includes the pre-identification of the translation of the at least one mRNA as rpL40-dependent, preferably to at least in part, more preferably to a substantial part thereof. A preferred tool for identifying such dependency uses the two-step specialized ribo-screen (2SSRS) as disclosed by, e.g. EP2251437, herewith incorporated by reference, which identifies target ribosomal proteins that tailor protein synthesis of mRNAs of proteins of interest (POIs). Comparative protein synthesis assays also identify mRNAs that are preferentially translated by distinct populations of specialized ribosomes. Furthermore, proteomic analysis can identify the absolute protein concentration in a given sample.

The protein rpL40 (either isolated or in combination with other ribosomal components) or a functional fragment thereof, as described herein, is then contacted (see above) with said at least one candidate compound in the presence of said at least one mRNA to be translated. Then, the modulation of the translation of said at least one mRNA compared to the translation in the absence of said at least one candidate compound is detected. Translation can be detected directly, for example by detecting the amount and/or presence and/or size (length) of the polypeptide as produced. This can be achieved with mass spectrometry, NMR, ELISA, labels that are include into the polypeptide, like labelled amino acids, luminescence constructs (renilla and/or firefly), fusions (GFP), and the like. Preferably, the detection involves a quantification of the translation product, and preferred is the method according to the present invention, wherein said modulation leads to or produces an increase or decrease of said rpL40-dependent translation of said at least one mRNA. Specific examples of modulation would be an increase of the translation of a polypeptide as produced, an inhibition (reduction) of polypeptides translated, or the amount or ratio (to each other) of polypeptides translated from a polycistronic mRNA.

Detecting the modulation of the translation of said at least one mRNA includes a detecting of whether a certain full-length or "correct" polypeptide has been made and/or whether a set of polypeptides has been made or not, and whether these translation products have been modulated in their sizes, amounts, and/or composition, as the case may require.

In support of the method according to the invention, small molecule binders and prospective modulators of rpL40 translation have been identified by a combination of bioinformatic studies, molecular docking studies, and in vitro NMR studies. The molecules as identified were the compound selected from the group of compounds selected from the following formulae: 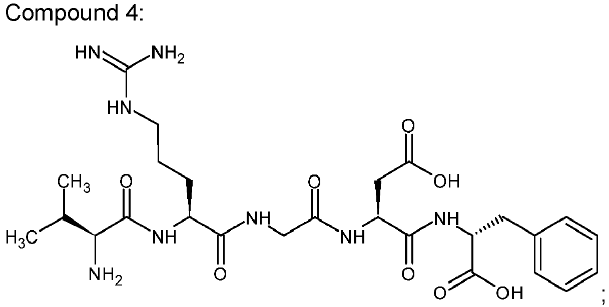 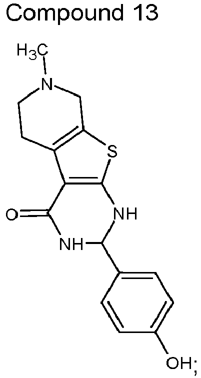 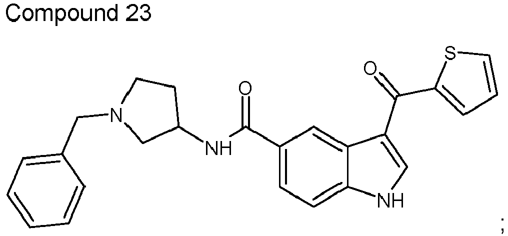 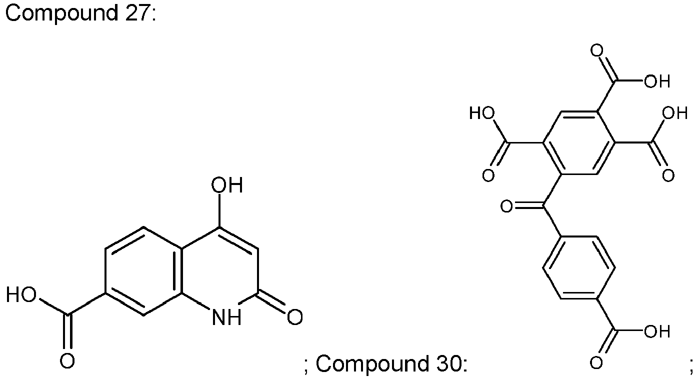 and

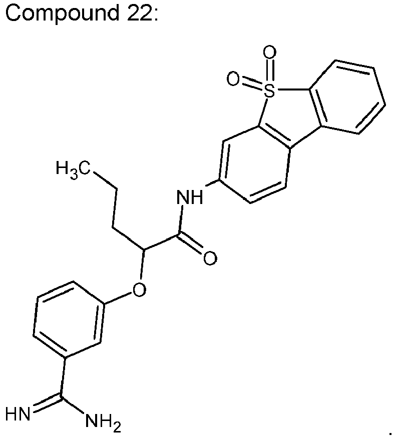

It is expected that derivatives and cosmetically and/or pharmaceutically acceptable salts of the compounds will show even improved properties to modulate rpL40 translation.

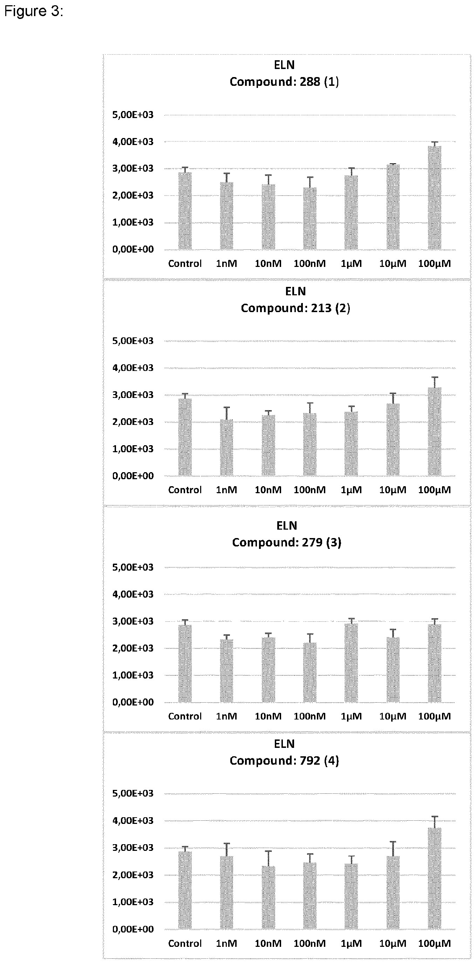

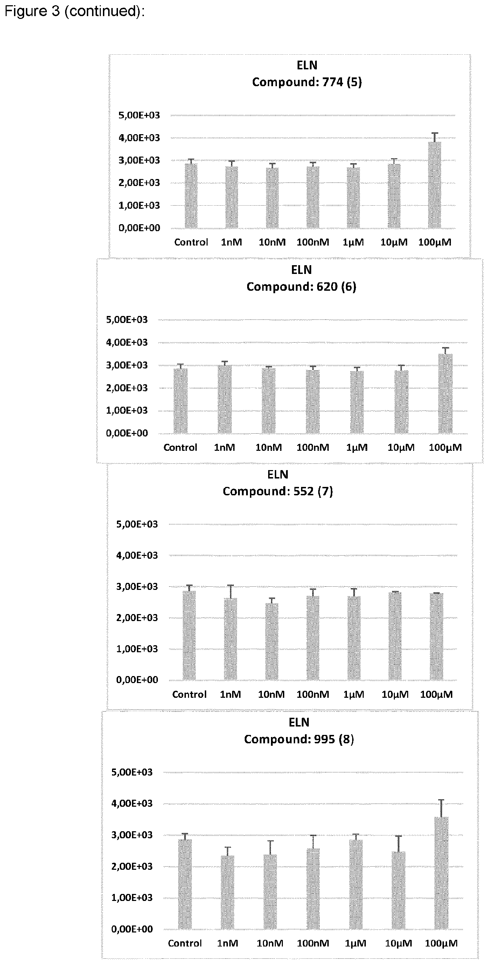

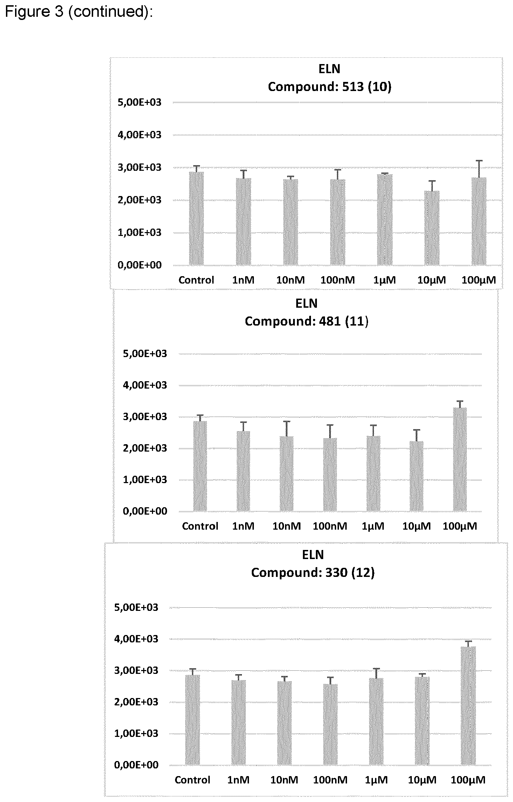

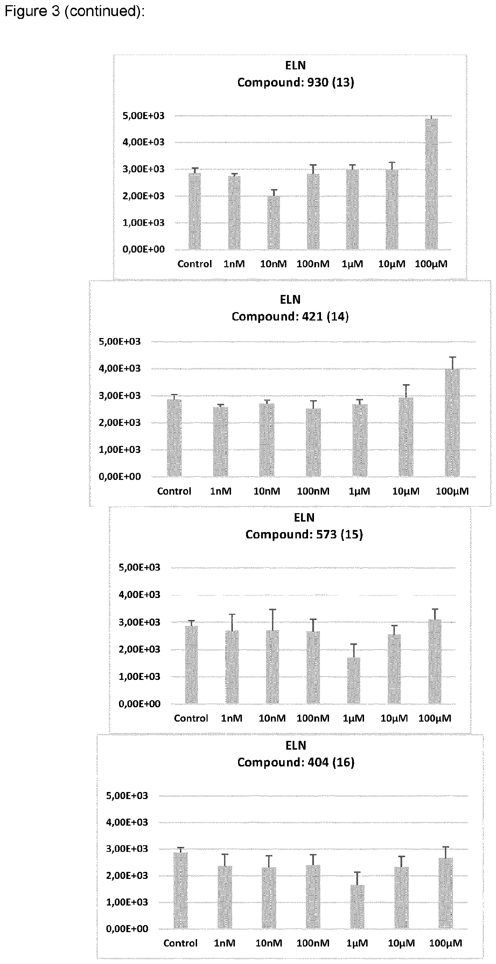

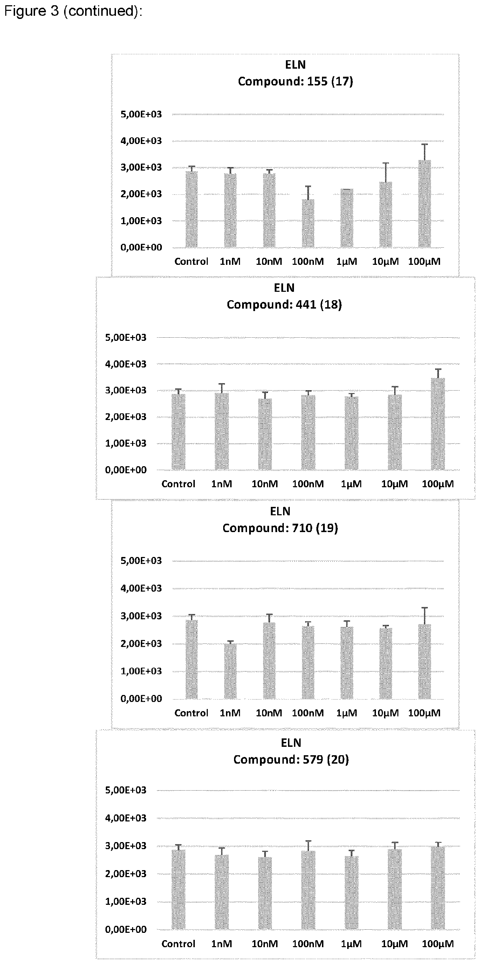

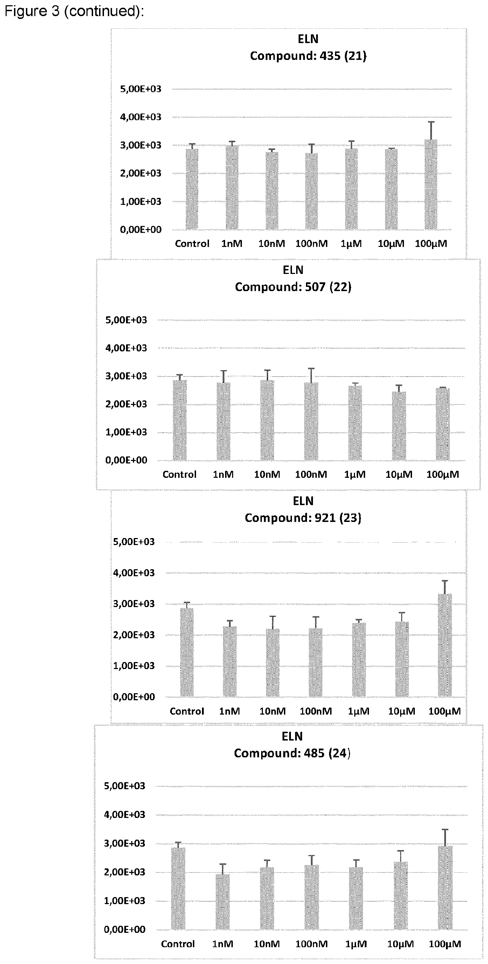

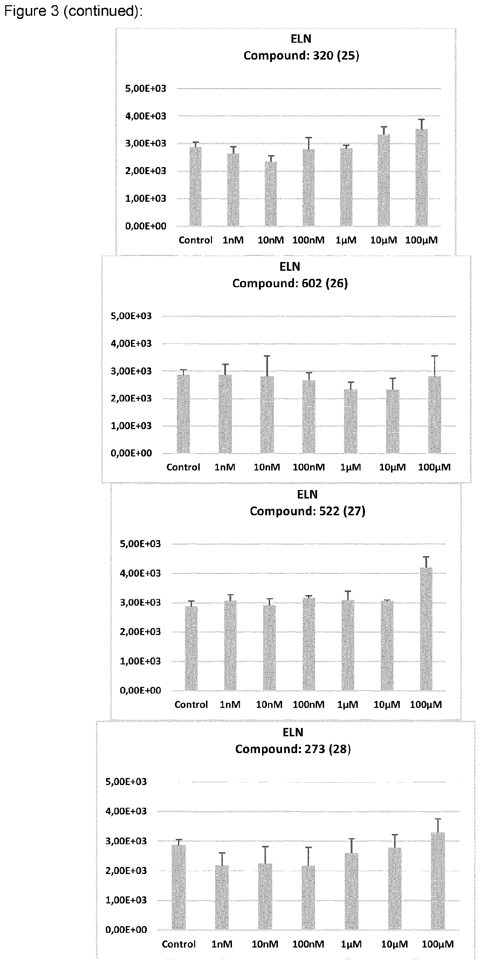

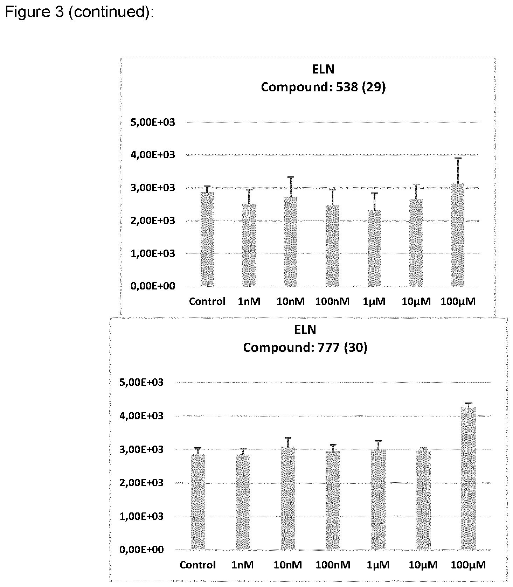

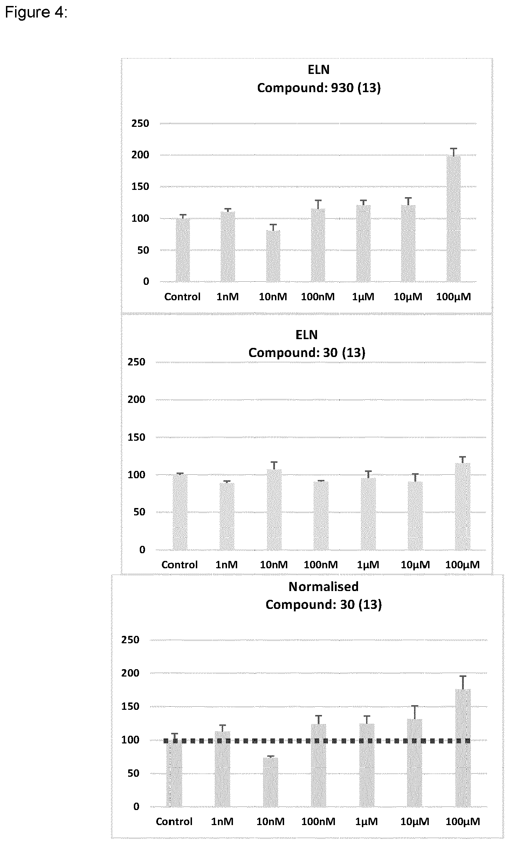

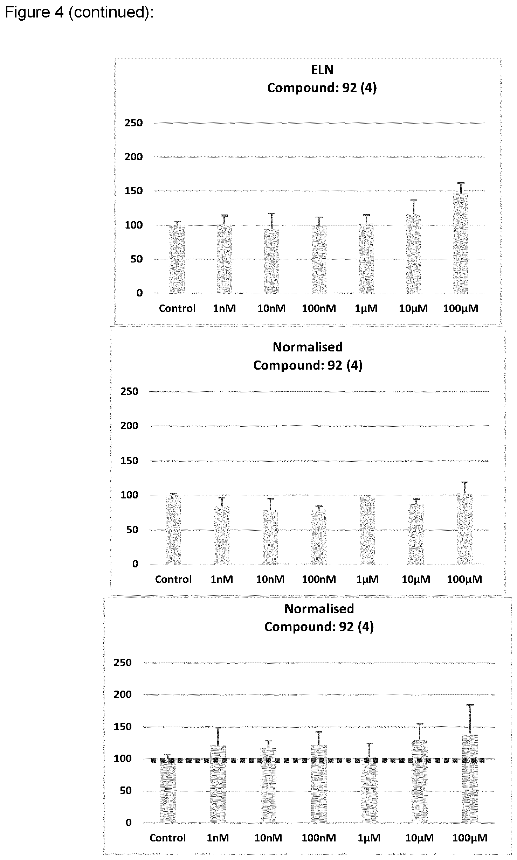

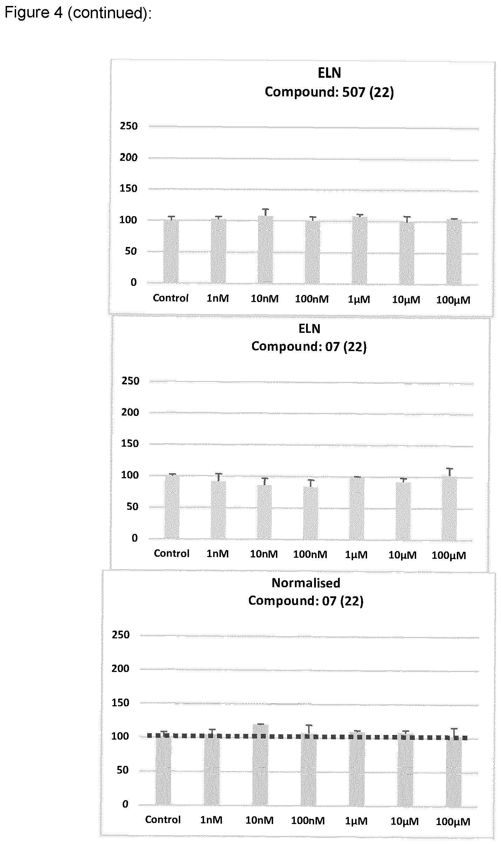

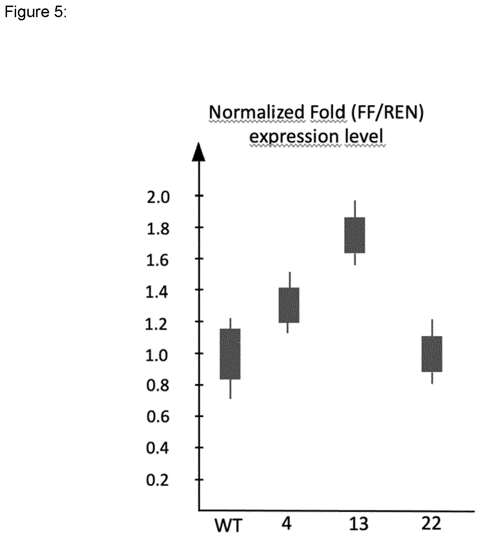

The inventors found that the compounds as identified, respectively, bind to human rpL40 (Figs. 3 to 6). The inventors further found that administration of the compounds increased translation in yeast cells for all compound except compound 22, which lowered translation (Fig. 3). The inventors further identified that the use of the compounds in combination even further improves, and in particular synergistically improves, the effect compared to either drug delivered alone.

Here, the inventors disclose human ribosomal protein rpL40 as a target for protein therapy to at least partially overcome and "repair" a lack of tropoelastin translation. The inventors show that in this way a treatment is achieved for the disorder, which is associated with the lack of tropoelastin translation/expression.

In the context of the present invention, the term "rpL40" shall include both the mammalian, preferably human, as well as the yeast protein or a functional fragment thereof. While the non-ubiquitinylated form is preferred, the term shall include both ubiquitinylated and non-ubiquitinylated forms. While the present invention ultimately aims at pharmaceutical compounds and compositions that are effective in a mammalian, such as a human patient, because of the conservation of the ribosomal proteins, the yeast system has constantly proven to be a valid model for the mammalian situation. Furthermore, the yeast system is more convenient to use as well. The term "rpL40" and/or "functional fragment" shall also include stretches and/or regions of the rpL40 polypeptide that are involved in the modulation of the translation of an mRNA. These areas are involved in binding of the compound (modulator) and/or a subsequent change of the mRNA translation, e.g. by steric hindrance or the promotion of the mRNA and/or polypeptide as produced by the ribosome. Preferred are functional fragments that include the C-terminal part of rpL40 as disclosed herein, i.e., between amino acids 77 and 128 as disclosed in SEQ ID NO: 2 or 4, and/or fragments facing the outside of the ribosome (resides on the surface of the ribosome exposed to the surrounding). The fragments can also be used/are useful for binding studies, either for the mRNA to be tested and/or for pre-screening of the modulator.

The amino acid sequence of human ubiquitin rpL40 is as follows:
MQIFVKTLTGKTITLEVEPSDTIENVKAKIQDKEGIPPDQQRLIFAGKQLEDGRTLSDYNIQ KESTLHLVLRLRGGIIEPSLRQLAQKYNCDKMICRKCYARLHPRAVNCRKKKCGHTNNLR PKKKVK (SEQ ID NO: 1). The amino acid sequence of relevant C-terminus (amino acids 77-128) of human rpL40 is as follows:

 (SEQ ID
NO: 2).

The amino acid sequence of yeast ubiquitin rpL40A is as follows:
MQIFVKTLTGKTITLEVESSDTIDNVKSKIQDKEGIPPDQQRLIFAGKQLEDGRTLSDYNIQ KESTLHLVLRLRGGIIEPSLKALASKYNCDKSVCRKCYARLPPRATNCRKRKCGHTNQLR PKKKLK (SEQ ID NO: 3). The amino acid sequence of relevant C-terminus (amino acids 77-128) of yeast rpL40 is as follows:

IIEPSLKALASKYNCDKSVCRKCYARLPPRATNCRKRKCGHTNQLRPKKKLK (SEQ ID
NO: 4).

Preferred is a method according to the present invention, furthermore comprising the step of detecting a binding of said at least one candidate compound to rpL40 or a fragment thereof, preferably to an isolated or partially isolated rpL40 or a fragment thereof, or to rpL40 in the context of the ribosomal subunit or in the context of both subunits of the mammalian ribosome. This aspect relates more to the situation *in vivo* and in the context of the complete ribosomal structure.

Preferred is also a method according to the present invention, furthermore comprising the step of detecting a binding of said at least one candidate compound to a fragment of rpL40, wherein said fragment comprises from about 30 to 90, preferably 60 to 80 of the C-terminal amino acids of the mammalian rpL40 protein, preferably according to SEQ ID NO: 2. This aspect relates more to the situation in with respect to the position(s) on rpL40 and the *in vitro* assays in the context of the present invention.

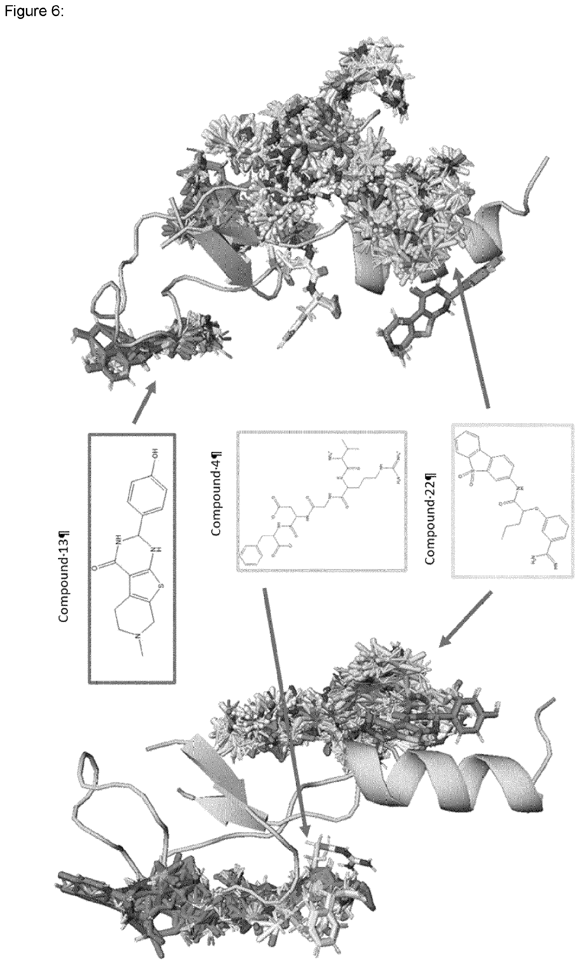

Further preferred is a method according to the present invention, wherein said detecting of binding (whether *in vivo* or *in vitro*) comprises detecting an interaction of said at least one candidate compound with an amino acid region of rpL40 selected from C-terminal from the last helix in the structure. This region of rpL40 was identified as being of particular relevance for the binding of compounds as identified, e.g., compounds 4 and 13, see also examples below and Figure 6.

Preferably, detecting of binding to rpL40 or the fragment thereof of the at least one candidate compound(s) is performed as a pre-screening before contacting said at least one candidate compound with said rpL40. That is, the present invention here includes a pre-selection based on the binding properties, e.g. on a recombinant rpL40, before including compounds in the more complex full assays.

Preferred is a method according to the present invention, furthermore, comprising a pre-selection step comprising molecular modeling of said binding of said at least one candidate compound to rpL40 or a fragment thereof. This can be done, for example, by using a computer program that identifies candidate compounds by molecular docking and structural analogs thereof, such as SwissDock. That is, the present invention here includes a pre-selection based on the binding properties as modelled *in silico,* e.g. based on the whole or a part of rpL40, either isolated or in the context of ribosomal proteins, before including compounds in the more complex full in vivo and/or vitro assays.

The above aspect regarding binding can be combined, if desired, e.g. the *in silico* results can be compared and validated with the *in vitro* results, and vice versa.

It is assumed in the context of the present invention, that binding of said at least one candidate compound to rpL40 or a fragment thereof constitutes an essential step for the modulation function of said compound. Nevertheless, the assays as described herein also includes pre-testing a binding in the presence or absence of the specific tropoelastin mRNA to be included.

Preferred is the method according to the present invention, wherein said rpL40 or fragment thereof is human rpL40. Comparative analysis of compound binding to yeast and human rpL40 showed that binding clusters overlap to a substantial degree, i.e., that on the level of *in silico* analysis the most prominent group of clusters of compounds bound to rpL40 are not essentially distinct for yeast and human. Thus, the yeast model system is regarded as sufficiently conserved in order to serve as tool for identifying the situation in human.

Further preferred is the method according to the present invention, wherein said method is performed *in vitro,* in cell culture or in vivo, preferably in a non-human mammal. More preferred is the combined assay of *in silico* binding with human *in vitro* cell culture assays, such as keratinocyte culture.

The candidate compound that is to be identified (screened) in the context of the present invention, can be any chemical substance or any mixture thereof. For example, it can be a substance of a peptide library, a library of small organic molecules, a combinatory library, a cell extract, in particular a plant cell extract, a "small molecular drug" (i.e. having a molecular weight of less than about 500 Da), a protein and/or a protein fragment, and an antibody or fragment thereof, and in particular from atazanavir and derivatives thereof and artesunate and derivatives thereof or combinations thereof. Plant extract libraries and "natural compounds" have proven to be of particular use. Particular examples are selected from the group of compounds selected from Compound 4, Compound 13, Compound 23, Compound 27, Compound 30, and Compound 22 as described before, and derivatives thereof, and cosmetically and/or pharmaceutically acceptable salts thereof.

Another aspect of the present invention then relates to a screening system for identifying a cosmetically and/or pharmaceutically active compound that modulates the rpL40-dependent translation of at least one mRNA encoding for a tropoelastin in a mammalian cell, comprising an eukaryotic cell recombinantly expressing a mammalian rpL40 or a fragment of a mammalian rpL40, wherein said fragment comprises from about 30 to 90, preferably 60 to 80 of the C-terminal amino acids of the mammalian rpL40 protein, preferably according to SEQ ID NO: 2, an expression construct for recombinantly expressing the at least one mRNA to be tested, and optionally, one or more candidate compounds to be tested.

The ribosomal protein rpL40 employed in the methods and systems of the present invention can be a full-length modified protein, e.g. including ubiquitin, or fragments with N/C -terminal and/or internal deletions. Preferably, the fragments are C-terminal non-ubiquitin fragments as above. Furthermore, the invention encompasses the use of mutated ribosomal proteins, such as proteins containing amino acid exchanges, modified amino acids, and fusion proteins. Methods for producing mutated ribosomal proteins are well known in the state of the art, and further described herein.

Preferred is a screening system according to the present invention that further comprises a recombinant expression construct, preferably an expression vector, for expressing the mammalian rpL40 or a fragment of a mammalian rpL40, preferably a human rpL40 or fragment thereof, and/or the (in this case proteinaceous or nucleic acid) at least one pharmaceutically active compound to be identified (screened).

Also preferred is a screening system according to the present invention wherein said expression construct for recombinantly expressing at least one mRNA to be tested further comprises at least one suitable reporter group, such as, for example, luciferase reporters. Most preferred is a dual luciferase reporter system, e.g., of luciferases from *Photinus pyralis* (firefly) and *Renilla reniformis.*

Basic ribosomal function and structural assembly of its components show a high degree of conservation throughout most biological kingdoms since about 3 billion years of evolution. It is preferred that the eukaryotic cells of the present invention might be selected from a large selection of different eukaryotic model systems, preferably selected from yeast or mammalian cells, such as mouse, rat, hamster (e.g. CHO), monkey or human cells, in particular keratinocytes. Not only mammalian cells might be preferred, but also invertebrate cells might be used for such a screening system, including for example insect cells. Preferred is a screening system according to the present invention wherein said eukaryotic cell is selected from a yeast, insect, hamster, or human cell.

Also preferred is a screening system according to the present invention wherein said eukaryotic cell is an inactivation or depletion mutant or comprises other modifications (e.g., posttranslational modifications) of rpL40. The inactivation, depletion or other modifications with respect to ribosomal protein rpL40 shall encompass all alterations (e.g., deletion or mutation) induced with techniques known to the skilled artisan that allow for the functional alteration (inactivation or reduced activity) of a ribosomal protein compared to its wild-type state, and/or the alteration of the expression level of said ribosomal protein or its respective mRNA. Enclosed are methods that interfere with appropriate protein function and/or expression at the level of genomic DNA, DNA transcription, mRNA stability and translation, protein expression and post- translational protein trafficking or protein modification. The present invention as an example embodiment thereof uses laboratory strains of cells, wherein the ribosomal protein gene for rpL40 (single and duplicated ribosomal protein genes encode for the 78 or 79 ribosomal proteins in yeast and mammalian cells, respectively) has been inactivated and/or depleted by deletion.

Another aspect of the present invention therefore relates to a compound that modulates the rpL40-dependent translation of at least one mRNA encoding for a tropoelastin in a mammalian cell for use in the prevention or treatment of diseases, in particular a disease or condition caused by a lack and/or insufficient synthesis of functional elastin, wherein said disease or condition is preferably selected from pulmonary hypertension, cigarette smoke induced emphysema, undesirable would healing, and severe chronic obstructive pulmonary disease (COPD), and wherein said compound was identified with a method according to the present invention, and in particular said compound is selected from Compound 4, Compound 13, Compound 23, Compound 27, Compound 30, and Compound 22 as described before, and derivatives thereof, and cosmetically and/or pharmaceutically acceptable salts thereof.

Thus, particularly preferred is a compound for use of the invention that modulates the rpL40-dependent translation of at least one tropoelastin mRNA in a mammalian cell which was identified with the enclosed screening systems and/or methods for screening. It is also preferred that the compound according to the present invention can be modified, for example chemically as described further below.

The compound for use and/or that is to be screened in the context of the present invention, can be any chemical substance or any mixture thereof. Preferably, said compound is selected from a chemical substance, a substance selected from a peptide library, a library of small organic molecules (i.e. of a molecular weight of about 500 Da or less), a combinatory library, a cell extract, in particular a plant cell extract, a small molecular drug, a protein and/or a protein fragment, and an antibody or fragment thereof, and in particular from the group of compounds selected from Compound 4, Compound 13, Compound 23, Compound 27, Compound 30, and Compound 22 as described before, and derivatives thereof, and cosmetically and/or pharmaceutically acceptable salts thereof.

In the context of the present invention, unless indicated otherwise, the term "about" shall mean +/- 10% of the value as given.

The selected or screened compound can then be modified. Said modification can take place in an additional preferred step of the methods of the invention as described herein, wherein, for example, after analyzing the translational activity of rpL40 or the fragment thereof in the presence and absence of said compound as selected, said compound is further chemically modified as described for example, below, and analyzed again for its effect on the translational activity of said ribosomal protein. Said "round of modification(s)" can be performed for one or several times in all the methods, in order to optimize the effect of the compound, for example, in order to improve its specificity for the target protein, and/or in order to improve its specificity for the specific tropoelastin mRNA translation to be influenced. This method is also termed "directed evolution" since it involves a multitude of steps including modification and selection, whereby binding compounds are selected in an "evolutionary" process optimizing its capabilities with respect to a particular property, e.g. its binding activity, its ability to activate, inhibit or modulate the activity, in particular the translational activity of the ribosomal protein rpL40 or the fragment(s) thereof.

The modification can also be simulated in silico before additional tests are performed in order to confirm or validate the effect of the modified selected or screened compound from the first round of screening. Respective software programs are known in the art and readily available for the person of skill.

Modification can further be effected by a variety of methods known in the art, which include without limitation the introduction of novel side chains or the exchange of functional groups like, for example, introduction of halogens, in particular F, Cl or Br, the introduction of lower alkyl groups, preferably having one to five carbon atoms like, for example, methyl, ethyl, n-propyl, isopropyl, n-butyl, isobutyl, tert-butyl, n-pentyl or isopentyl groups, lower alkenyl groups, preferably having two to five carbon atoms, lower alkynyl groups, preferably having two to five carbon atoms or through the introduction of, for example, a group selected from the group consisting of NH₂, NO₂, OH, SH, NH, CN, aryl, heteroaryl, COH or COOH group.

Yet another important aspect of the present invention then relates to a method for manufacturing a cosmetic and/or pharmaceutical composition for modulating the rpL40-dependent translation of at least one mRNA encoding for a tropoelastin in a mammalian cell, comprising the steps of formulating the compound according to the present invention into a suitable pharmaceutical and/or cosmetic composition, or performing a method according to the present invention for identifying a compound that modulates the rpL40-dependent translation of at least one mRNA in a mammalian cell, and formulating said compound as identified into a suitable pharmaceutical and/or cosmetic composition. The invention also relates to a cosmetic and/or pharmaceutical composition obtained by said method according to the present invention. Preferably, said modulating the rpL40-dependent translation of at least one mRNA encoding for a tropoelastin is for preventing or treating a condition selected from photo ageing, skin rejuvenation, and increasing functional elastin synthesis. Preferably, said disease or condition is selected from a disease or condition caused by a lack and/or insufficient synthesis of functional elastin, wherein said disease or condition is preferably selected from pulmonary hypertension, cigarette smoke induced emphysema, and severe chronic obstructive pulmonary disease (COPD), or treating or preventing senescent cells.

Thus, in yet another aspect of the present invention, the selected or screened compound and/or compound for use can be provided and/or is administered as a suitable pharmaceutical composition, such as a topical composition, tablet, capsule, granule, powder, sachet, reconstitutable powder, dry powder inhaler and/or chewable. Such solid formulations may comprise excipients and other ingredients in suitable amounts. Such solid formulations may contain e.g., cellulose, cellulose microcrystalline, polyvidone, in particular FB polyvidone, magnesium stearate and the like. The interacting compound identified as outlined above, which may or may not have gone through additional rounds of modification, is admixed with suitable auxiliary substances and/or additives. Such substances comprise pharmacological acceptable substances, which increase the stability, solubility, biocompatibility, or biological half-life of the interacting compound or comprise substances or materials, which must be included for certain routes of application like, for example, intravenous solution, sprays, liposomes, ointments, skin creme, band-aids or pills.

It is to be understood that the present compound and/or a pharmaceutical composition comprising the present compound is for use to be administered to a human patient. The term "administering" means administration of a sole therapeutic agent or in combination with another therapeutic agent. It is thus envisaged that the pharmaceutical composition of the present invention are employed in co-therapy approaches, i.e. in co-administration with another, or other medicaments or drugs and/or any other therapeutic agent which might be beneficial in the context of the methods of the present invention. Nevertheless, the other pharmaceutical composition of the present invention, medicaments or drugs and/or any other therapeutic agent can be administered separately from the compound as selected or screened and/or compound for use, if required, as long as they act in combination (i.e., directly and/or indirectly, preferably synergistically) with the present compound as selected or screened and/or for use.

Thus, the compounds as selected or screened and/or for use of the invention can be used alone or in combination with other active compounds - for example with medicaments already known for the treatment of the aforementioned diseases, whereby in the latter case a favorable additive, amplifying or preferably synergistically effect is noticed. Suitable amounts to be administered to humans range from 5 to 500 mg, in particular 10 mg to 100 mg. Of course, any dosage can be readily adjusted by the attending physician, if needed, based on, for example, other medical parameters of the patient to be treated.

Pharmaceutical compositions as used may optionally comprise a pharmaceutically acceptable carrier. Pharmaceutically acceptable carriers or excipients include diluents (fillers, bulking agents, e.g. lactose, microcrystalline cellulose), disintegrants (e.g. sodium starch glycolate, croscarmellose sodium), binders (e.g. PVP, HPMC), lubricants (e.g. magnesium stearate), glidants (e.g. colloidal SiO2), solvents/co-solvents (e.g. aqueous vehicle, Propylene glycol, glycerol), buffering agents (e.g. citrate, gluconates, lactates), preservatives (e.g. Na benzoate, parabens (Me, Pr and Bu), BKC), anti -oxidants (e.g. BHT, BHA, Ascorbic acid), wetting agents (e.g. polysorbates, sorbitan esters), thickening agents (e.g. methylcellulose or hydroxyethylcellulose), sweetening agents (e.g. sorbitol, saccharin, aspartame, acesulfame), flavoring agents (e.g. peppermint, lemon oils, butterscotch, etc.), humectants (e.g. propylene, glycol, glycerol, sorbitol). Other suitable pharmaceutically acceptable excipients are inter alia described in Remington's Pharmaceutical Sciences, 15th Ed., Mack Publishing Co., New Jersey (1991) and Bauer et al., Pharmazeutische Technologic, 5th Ed., Govi-Verlag Frankfurt (1997). The person skilled in the art knows suitable formulations for respective compounds, for example topical, and will readily be able to choose suitable pharmaceutically acceptable carriers or excipients, depending, e.g., on the formulation and administration route of the pharmaceutical composition.

The therapeutics can be administered orally, e.g., in the form of pills, tablets, coated tablets, sugar coated tablets, hard and soft gelatin capsules, solutions, syrups, emulsions or suspensions or as aerosol mixtures. Administration, however, can also be carried out rectally, e.g., in the form of suppositories, or parenterally, e.g., in the form of injections or infusions, or percutaneously, e.g., in the form of ointments, creams or tinctures.

In addition to the aforementioned compounds as selected or screened and/or for use of the invention, the pharmaceutical composition can contain further customary, usually inert carrier materials or excipients. Thus, the pharmaceutical preparations can also contain additives, such as, for example, fillers, extenders, disintegrants, binders, glidants, wetting agents, stabilizers, emulsifiers, preservatives, sweetening agents, colorants, flavorings or aromatizers, buffer substances, and furthermore solvents or solubilizers or agents for achieving a depot effect, as well as salts for changing the osmotic pressure, coating agents or antioxidants. They can also contain the aforementioned salts of two or more compounds for use of the invention and also other therapeutically active substances as described above.

Yet another aspect of the present invention then relates to a method, in particular a cosmetic or non-medical method, for modulating the rpL40-dependent translation of at least one mRNA encoding for a tropoelastin in a mammalian cell or tissue, comprising contacting said cell or tissue with an effective amount of a compound that was identified with a method according to the present invention and/or for use according to the present invention, or the cosmetic and/or pharmaceutical composition according to the present invention, preferably selected from the group of compounds selected from Compound 4, Compound 13, Compound 23, Compound 27, Compound 30, and Compound 22 as described before, and derivatives thereof, and cosmetically and/or pharmaceutically acceptable salts thereof.

Preferably, said method is a non-medical or cosmetic method, and/or is performed *in vivo* or *in vitro.* Further preferably, said modulating the rpL40-dependent translation of at least one mRNA encoding for a tropoelastin is for preventing or treating a condition selected from photo ageing, skin rejuvenation, and increasing functional elastin synthesis.

Yet another important aspect of the present invention then relates to a method of preventing, treating or ameliorating a disease or condition caused by a lack and/or insufficient synthesis of functional elastin, wherein said disease or condition is preferably selected from pulmonary hypertension, cigarette smoke induced emphysema, and severe chronic obstructive pulmonary disease (COPD), comprising administering an effective amount of a compound that was identified with a method according to the present invention, or of the pharmaceutical composition according to the present invention to a patient or subject in need of said treatment or prevention, thereby treating, preventing or ameliorating a disease or condition

By "treatment" or "treating" is meant any treatment of a disease or disorder, in a mammal, including preventing or protecting against the disease or disorder, that is, causing, the clinical symptoms of the disease not to develop; inhibiting the disease, that is, arresting or suppressing the development of clinical symptoms; and/or relieving the disease, that is, causing the regression of clinical symptoms. By "amelioration" is meant the prevention, reduction or palliation of a state, or improvement of the state of a subject; the amelioration of a stress is the counteracting of the negative aspects of a stress. Amelioration includes, but does not require, complete recovery or complete prevention of a stress.

As above, the compound as administered in the context of the present invention can be any chemical substance or any mixture thereof. Preferably, said compound is selected from a substance selected from a peptide library, a library of small organic molecules (i.e., of a molecular weight of about 500 Da or less), a combinatory library, a cell extract, in particular a plant cell extract, a small molecular drug, a protein and/or a protein fragment, and an antibody or fragment thereof, and in particular selected from the group of compounds selected from Compound 4, Compound 13, Compound 23, Compound 27, Compound 30, and Compound 22 as described before, and derivatives thereof, and cosmetically and/or pharmaceutically acceptable salts thereof.

Preferred is a method according to the present invention, wherein said disease or condition is selected from pulmonary hypertension, cigarette smoke induced emphysema, and severe chronic obstructive pulmonary disease (COPD).

In this context it is important to note that in many elastin-associated disease states or conditions a relatively small increase in a protein translation from the targeted mRNA (e.g., by increasing stability of the targeted mRNA), such a 2- to 2.5-fold is deemed sufficient for a significant improvement of said disease state or condition. In other cases, in turn, it may be desirable to reduce the amount of a target protein, preferably to a minimal level, such as, for example, to 50% or less, or 25% or less, compared to the wild-type.

The inventors used the Riboscreen^{®} technology and 2SSRS screening tool (see above) to identify possible ribosomal protein targets which might serve as ribosomal drug targets specific for systemic repair of insufficient tropoelastin production.

In this way, the inventors identified ribosomal protein rpL40 as a target ribosomal protein (TRP) for customized increase in protein expression of tropoelastin with no observable effect on the altered production of other ("bulk") mRNAs. The yeast ribosomal protein rpL40 is a structural homologue of the human rpL40 and occupies the identical position on the yeast and human ribosome.

In the context of the present invention, the inventors have identified several candidate small molecule binders of yeast and human rpL40. First, the inventors employed molecular docking tools to probe binding sites of the compounds on yeast rpL40 and human rpL40, respectively. The in-silico models indicated overlapping binding clusters of the molecules, in particular compounds 4 and 13. These findings encouraged a more detailed in vitro analysis. Small molecule human rpL40 interaction was assessed by NMR titration series. Analysis of changes in the NMR spectra in the presence of small molecules as identified demonstrated that rpL40 candidate ligands bind human rpL40 protein at several possible binding pockets. The binding epitope was narrowed down to the C-terminal site, i.e., the non-ubiquitin part of amino acids 77 to 128, the more C-terminal site. Combining the results from NMR titration analysis, bioinformatic docking studies on yeast and human rpL40 and spatial arrangement of rpL40 in the ribosome, the inventors conclude that the most favorable binding site for both compounds is the C-terminal site epitope formed by amino acids behind the helix (see Fig. 6). Inspection of rpL40 on the ribosome suggests that for rpL40 in its natural state and integrated into the ribosome, the relevant C-terminal site epitope is accessible to the compounds as tested (see also above for "fragments").

The inventors conclude that the interaction of the compounds as identified with human rpL40 as identified here demonstrates the power of the 2SSRS screen to identify a target ribosomal protein rpL40 and candidate drugs interacting with rpL40 in such a way that customized boost in production of full-length tropoelastin protein. It sets the stage for the functional analysis of these compounds in yeast cells, in human HeLa cell extracts and in general in human cells, such as keratinocytes. In the inventors' case, these experiments show the potential of small molecule rpL40 ligands, respectively, to act as a molecular switch for rpL40 to customize increased production the tropoelastin protein, in particular tropoelastin isoform VI.

In the context of the present invention, the inventors explored a novel strategy to alter endogenously protein production levels of tropoelastin by targeting the protein synthesis machinery, the ribosome. The translating ribosome is assembled from two unequal subunits, each composed of ribosomal RNA (rRNA) and ribosomal proteins (RPs). The complex eukaryotic ribosome consists of a large 60S subunit harboring 50 ribosomal proteins and a small 40S subunit, harboring 30 ribosomal proteins (Spahn CM, Beckmann R, Eswar N, Penczek PA, Sali A, Blobel G, Frank J. Structure of the 80S ribosome from Saccharomyces cerevisiae--tRNA-ribosome and subunit-subunit interactions. Cell. 2001 Nov 2;107(3):373-86. doi: 10.1016/s0092-8674(01)00539-6. PMID: 11701127). While during eukaryotic evolution the rRNA tracts have been expanded, the 80 ribosomal proteins of the cytoplasmic ribosome have been highly conserved in sequence, structure and function. Ribosomes are primed for mRNA translation by complex initiation processes which generate the translationally competent ribosome, which then decodes mRNA into proteins. For several decades, it has been thought that during this process, all ribosomes serve as static translation platforms, receiving regulatory input primarily from both general and mRNA-specific translation factors (Sonenberg N, Hinnebusch AG. Regulation of translation initiation in eukaryotes: mechanisms and biological targets. Cell. 2009 Feb 20;136(4):731-45. doi: 10.1016/j.cell.2009.01.042. PMID: 19239892; PMCID: PMC3610329). However, several lines of evidence now point to the possibility that sub-populations of ribosomes - heterologous or "specialized" ribosomes with intrinsically altered translational activity - may actually exist in a cell as well.

Such "specialized" ribosomes are thought to favor altered protein production levels of a subpopulation of mRNAs, while leaving bulk protein synthesis unaltered. Specialized ribosomes have been recently engineered in human embryonic stem cells to monitor the effect of depletion of ribosomal proteins on generating selective protein signatures. (Shi Z, Fujii K, Kovary KM, Genuth NR, Röst HL, Teruel MN, Barna M. Heterogeneous Ribosomes Preferentially Translate Distinct Subpools of mRNAs Genome-wide. Mol Cell. 2017 Jul 6;67(1):71-83.e7. doi: 10.1016/j.molcel.2017.05.021. Epub 2017 Jun 15. PMID: 28625553; PMCID: PMC5548184).

As strategies to specialize ribosomes modifications of the ribosomal constituents, the ribosomal proteins, are of particular interest, and their high structural and functional conservation during eukaryotic evolution allows for development of cost-efficient screening in yeast vehicles. Therefore, yeast *Saccharomyces cerevisiae* ribosome serves as a prototype for exploiting natural strategies to engineer specialized ribosomes. As mentioned above, the inventors used the Riboscreen^{®} technology (Bauer JW, Brandl C, Haubenreisser O, Wimmer B, Weber M, Karl T, Klausegger A, Breitenbach M, Hintner H, von der Haar T, Tuite MF, Breitenbach-Koller L. Specialized yeast ribosomes: a customized tool for selective mRNA translation. PLoS One. 2013 Jul 8;8(7):e67609. doi: 10.1371/journal.pone.0067609. PMID: 23861776; PMCID: PMC3704640) to screen a large collection of diploid yeast strains, each deficient in one or other copy of the set of ribosomal proteins (RP) genes and constituting a variant ribosomal protein (VRP) strain collection, to generate eukaryotic cells carrying distinct sub-populations of altered 'specialized' ribosomes.

Such specialized ribosomes are characterized by the functional and/or physical absence of a (one) RP, which serves as proxy for altered functional availability of this RP. Using a heterologous tropoelastin reporter tagged with firefly (FF) luciferase and an independently expressed renilla luciferase (REN), the question was addressed which of the VRP strains could carry a sub-population of ribosomes characterized by an altered functional availability of a ribosomal protein with relevance for the translation of tropoelastin. Once identified, such a ribosomal protein in vivo may then serve as a target ribosomal protein (TRP) in order to identify small molecule ligands for a systemic treatment, again targeting translation of tropoelastin.

One advantage of the strategy of using specialized ribosomes is the identification of a druggable target protein that is specific for the desired increase/decrease in translation of a protein, here tropoelastin. Once a target protein, here rpL40, is identified, a suite of drug screening regimes can be employed for the discovery of a molecular modulator of rpL40 in the second step of the Riboscreen^{®} that mimics the depletion of the rpL40 in the first step of the screening.

Ribsosomal proteins were only recently identified as prospective candidate regulators of selected mRNA translations, because for decades it was thought that they would have a primarily structural role in ribsosome function. However, biochemical studies have now shown that altering the functional availability of ribosomal proteins has the potential to edit production levels of subsets of mRNAs, while leaving the bulk translation unaltered. Importantly, targeting ribosomal proteins is a fine-tuned instrument for changing production levels of a protein of interest, as to date there are no reports that such an intervention might increase or decrease production levels more than several fold.

Therefore, this approach provides a narrow but physiologically acceptable corridor of changes in production level of proteins that does not come in conflict with overall proteome homeostasis and ensures that protein flux of the cell remains in balance to provide proper overall cell function (Harper JW, Bennett EJ. Proteome complexity and the forces that drive proteome imbalance. Nature. 2016 Sep 15;537(7620):328-38. doi: 10.1038/nature19947. PMID: 27629639; PMCID: PMC5204264).

The technology as used identified ribosomal protein L40 as a ribosomal target to boost (increase) production levels of tropoelastin at about 1.5 to 2.5-fold. For aging skin cells to be treated, small molecule ligands of RPI40 have been identified and tested for their ability to increase production levels of tropoelastin in cellular assays.

The present invention preferably relates to the following items.

Item 1. A method for identifying a cosmetically and/or pharmaceutically active compound that modulates the rpL40-dependent translation of at least one mRNA encoding for a tropoelastin in a mammalian cell, comprising
a) contacting rpL40 or a functional fragment thereof with at least one candidate compound in the presence of said at least one mRNA to be translated, and
b) detecting the modulation of the translation of said at least one mRNA encoding for a tropoelastin compared to the translation in the absence of said at least one candidate compound, wherein a modulation of the translation of said at least one mRNA is indicative for a cosmetically and/or pharmaceutically active compound.

Item 2. The method according to Item 1, furthermore comprising the step of a pre-identifying the translation of said at least one mRNA as being rpL40-dependent.

Item 3. The method according to Item 1 or 2, wherein said modulation leads to an increase or decrease of said rpL40-dependent translation of said at least one mRNA.

Item 4. The method according to any one of Items 1 to 3, furthermore comprising detecting a binding of said at least one candidate compound to rpL40, preferably to an isolated or partially isolated rpL40, or to rpL40 in the context of the ribosomal subunit or in the context of both subunits of the mammalian ribosome.

Item 5. The method according to any one of Items 1 to 4, furthermore comprising detecting a binding of said at least one candidate compound to a fragment of rpL40, wherein said fragment comprises from about 30 to 90, preferably 60 to 80 of the C-terminal amino acids of the mammalian rpL40 protein, preferably according to SEQ ID NO: 2.

Item 6. The method according to any one of Items 1 to 5, wherein said detecting of binding to rpL40 or the fragment thereof is performed as a pre-screening before contacting said at least one candidate compound with said rpL40.

Item 7. The method according to any one of Items 1 to 6, furthermore comprising a pre-selection step comprising molecular modeling of said binding of said at least one candidate compound to rpL40 or a fragment thereof, for example using a computer program, such as SwissDock.

Item 8. The method according to any one of Items 1 to 7, wherein said rpL40 or fragment thereof is human rpL40.

Item 9. The method according to any one of Items 1 to 8, wherein said method is performed in vitro, in cell culture or in vivo, preferably in a non-human mammal.

Item 10. The method according to any one of Items 1 to 9, wherein said candidate compound is selected from a chemical substance, a substance selected from a peptide library, a library of small organic molecules, a combinatory library, a cell extract, in particular a plant cell extract, a "small molecular drug", a natural compound, a protein and/or a protein fragment, and an antibody or fragment thereof, and in particular from the group of compounds selected from Compound 4, Compound 13, Compound 23, Compound 27, Compound 30, and Compound 22 as described before, and derivatives thereof, and cosmetically and/or pharmaceutically acceptable salts thereof.

Item 11. The method according to any one of Items 1 to 10, wherein said at least one mRNA encodes for a tropoelastin isoform, in particular tropoelastin isoform VI.

Item 12. A screening system for identifying a cosmetically and/or pharmaceutically active compound that modulates the rpL40-dependent translation of at least one mRNA encoding for a tropoelastin in a mammalian cell, comprising
an eukaryotic cell recombinantly expressing a mammalian rpL40 or a fragment of a mammalian rpL40, wherein said fragment comprises from about 30 to 90, preferably 60 to 80 of the C-terminal amino acids of the mammalian rpL40 protein, preferably according to SEQ ID NO: 2,
an expression construct for recombinantly expressing the at least one mRNA to be tested, and
optionally, one or more candidate compounds to be tested.

Item 13. The screening system according to Item 12, wherein said eukaryotic cell is selected from a yeast, insect, rodent, or human cell.

Item 14. The screening system according to Item 12 or 13, wherein said eukaryotic cell is an inactivation or depletion mutant of rpL40.

Item 15. A compound that modulates the rpL40-dependent translation of at least one mRNA encoding for a tropoelastin in a mammalian cell for use in the prevention or treatment of diseases, in particular a disease or condition caused by a lack and/or insufficient synthesis of functional elastin, wherein said disease or condition is preferably selected from pulmonary hypertension, cigarette smoke induced emphysema, and severe chronic obstructive pulmonary disease (COPD), and wherein said compound was identified with a method according to any one of Items 1 to 11.

Item 16. A cosmetic and/or pharmaceutical composition comprising at least one compound modulating the rpL40-dependent translation of at least one mRNA encoding for a tropoelastin in a mammalian cell according to Item 15, together with at least one cosmetically and/or pharmaceutically acceptable carrier, preferably for systemic or topical administration.

Item 17. A method, in particular a cosmetic or non-medical method, for modulating the rpL40-dependent translation of at least one mRNA encoding for a tropoelastin in a mammalian cell or tissue, comprising contacting said cell or tissue with an effective amount of a compound that was identified with a method according to any one of Items 1 to 11, or the cosmetic and/or pharmaceutical composition according to Item 16.

Item 18. The method according to Item 17, wherein said modulating the rpL40-dependent translation of at least one mRNA encoding for a tropoelastin is for preventing or treating a condition selected from photo ageing, skin rejuvenation, and increasing functional elastin synthesis.

Item 19. A method of preventing, treating or ameliorating a disease or condition caused by a lack and/or insufficient synthesis of functional elastin, wherein said disease or condition is preferably selected from pulmonary hypertension, cigarette smoke induced emphysema, and severe chronic obstructive pulmonary disease (COPD), comprising administering an effective amount of a compound that was identified with a method according to any one of Items 1 to 11, or of the pharmaceutical composition according to Item 16 to a patient or subject in need of said treatment or prevention.

The present invention will now be described further in the following examples with reference to the accompanying Figures, nevertheless, without being limited thereto. For the purposes of the present invention, all references as cited herein are incorporated by reference in their entireties.
Figure 1 shows a schematic overview of the variants of tropoelastin.
Figure 2 shows the correlation of TE-FF/REN luciferase reporter expression by variant diploid yeast strains. The wildtype (abbreviated WT) is shown as a red dot. RP deletion strain RPL40A/delRPL40A with increased TE-FF expression, and RP deletion strain RPS26A/delRPS26A with unspecific increase of TE-FF and REN expression are marked.
Figure 3 shows result for the assays testing the biological activity of prospective L40 binders -assessment of 29 candidate L40 ligands. Replicates n=3, and treatment was made at 1nM, 10nM, 100nm, 1µM, 10µM, and 100µM concentration of compounds. Boxes indicate active compounds.
Figure 4 shows the results for an additional analysis of the biological activity of rpL40 binders compounds 4 and 13, and compound 22. Replicates n=3, and treatment was made at 1nM, 10nM, 100nm, 1µM, 10µM, and 100µM concentration of compounds.
Figure 5 shows a summary of the results of the additional analysis of the biological activity of rpL40 binders compounds 4 and 13, as well as compound 22 decreasing translation.
Figure 6 shows the results of an *in silico* analysis of binding sites of the compounds 4, 13, and 22 on the structure of RPL40 (yeast). Notably, compounds 4 and 13 bind at a defined region at the C-terminus, whereas compound 22 does not. Compounds 4, 13, and 22 bind to another cluster region, further C-terminal.

### Examples

The following examples relate to tropoelastin isoform VI as a protein of interest (POI) and the ribosomal protein rpL40 as the target ribosomal protein. Nevertheless, as the person of skill will be aware, tropoelastin isoform VI is only a preferred example, and other elastin components and/or isoforms can be readily identified and tested by the person of skill based on and similar to the examples as given herein, without the need for any substantial modification of the assays as disclosed.

### Materials and Methods

### Yeast strains, E. coli strains, growth media

In the yeast *S*. *cerevisiae,* the 78 RPs are encoded by 137 genes that include 19 single copy RP genes and 59 duplicated RP genes. Diploid yeast strains, each deficient in one or other copy of the set of ribosomal protein (RP) genes, and thus creating eukaryotic cells carrying distinct populations of altered 'specialized' ribosomes, were used as described in Bauer et al. (in: Specialized yeast ribosomes: a customized tool for selective mRNA translation. PLoS One. 2013; 8(7):e67609. doi:10.1371/journal.pone.0067609) and EP2251437, which are herewith both incorporated by reference in their entireties.

Yeast and *E. coli* media, culture conditions and the manipulation of yeast and E. coli strains were as described previously (Oender K, Loeffler M, Doppler E, Eder M, Lach S, Heinrich F, Karl T, Moesl R, Hundsberger H, Klade T, Eckl P, Dickinson JR, Breitenbach M, Koller L. Translational regulator RpL10p/Grc5p interacts physically and functionally with Sed1p, a dynamic component of the yeast cell surface. Yeast. 2003 Mar;20(4):281-94. doi: 10.1002/yea.963. PMID: 12627396). The RP deletion strains as used were grown on yeast extract - peptone - dextrose (YPD) medium or defined YNB-based medium (SC) to provide the desired selective conditions.

### Cloning of the tropoelastin-firefly protein expression reporter

Reporter parent vectors used were the yeast centromeric plasmids YCplac33 (URA3) and YCplac111 (LEU2) (Bauer JW, Brandl C, Haubenreisser O, Wimmer B, Weber M, Karl T, Klausegger A, Breitenbach M, Hintner H, von der Haar T, Tuite MF, Breitenbach-Koller L. Specialized yeast ribosomes: a customized tool for selective mRNA translation. PLoS One. 2013 Jul 8;8(7):e67609. doi: 10.1371/journal.pone.0067609. PMID: 23861776; PMCID: PMC3704640). To generate the Tropoelastin-FF Luciferase reporter, first the tropoelastin sequence (tropoelastin isoform VI) was PCR amplified from parent vector pCMV6-XL6_ELN-lso-VI, using forward primer F 5'-GGACTCTAGAGCGGGTCTGACGGCGGC-3' (SEQ ID NO: 5) and reverse primer R 5'-CCTGGGTACCTTTTCTCTTCCGGCCACAAG-3' (SEQ ID NO: 6), containing restriction sites for Xbal at the 5' and Kpnl at the 3' end. A previously generated YCpLac33_LAMB3-PTC_FF plasmid (Bauer JW, Brandl C, Haubenreisser O, Wimmer B, Weber M, Karl T, Klausegger A, Breitenbach M, Hintner H, von der Haar T, Tuite MF, Breitenbach-Koller L.

Specialized yeast ribosomes: a customized tool for selective mRNA translation. PLoS One. 2013 Jul 8;8(7):e67609. doi: 10.1371/journal.pone.0067609. PMID: 23861776; PMCID: PMC3704640), which was generated to express the reporter sequence under control of ADH1 promotor and terminator sequences, respectively, was digested with FastDigest Xbal (ThermoScientific) and FastDigest Kpnl (ThermoScientific) in order to eliminate the LAMB3-PTC sequence and to replace it with the tropoelastin sequence. This provided the TE_FF reporter. Correct integration of the tropoelastin sequence was verified by sequencing and control digest. The renilla (REN) protein expression reporter, also under the control of the ADH1 promotor and ADH1 terminator, respectively, was available from previous studies (Bauer JW, Brandl C, Haubenreisser O, Wimmer B, Weber M, Karl T, Klausegger A, Breitenbach M, Hintner H, von der Haar T, Tuite MF, Breitenbach-Koller L. Specialized yeast ribosomes: a customized tool for selective mRNA translation. PLoS One. 2013 Jul 8;8(7):e67609. doi: 10.1371/journal.pone.0067609. PMID: 23861776; PMCID: PMC3704640) and contains the renilla sequence in a YCpLac111 parent vector (Gietz RD, Sugino A. New yeast-Escherichia coli shuttle vectors constructed with in vitro mutagenized yeast genes lacking six-base pair restriction sites. Gene. 1988 Dec 30;74(2):527-34. doi: 10.1016/0378-1119(88)90185-0. PMID: 3073106).

### Dual Luciferase assay

Wildtype (BY2n) and variant ribosomal protein yeast strains (Kelly DE, Lamb DC, Kelly SL. Genome-wide generation of yeast gene deletion strains. Comp Funct Genomics. 2001;2(4):236-42. doi: 10.1002/cfg.95. PMID: 18628917; PMCID: PMC2447215), that were co-transformed with the tropoelastin-firefly and the renilla protein expression reporter plasmids were used for overnight cultures using single colony inoculation. Cell density (OD600) was determined using a Hitachi U_3000 spectrophotometer. Erlenmeyer flasks containing 13 ml SC-URA-LEU medium were inoculated with at OD600 = 0.065. Cultures were grown overnight at 28°C under constant shaking, and then harvested near exponential phase at OD600 = 1.8 to 2.0 with centrifugation, and the pellet was diluted to 10⁷ cells per ml in autoclaved water.

Dual luciferase assay readouts were performed with the GloMax Multi Detection System using the Dual-Luciferase^{®} Reporter Assay System (Promega). For each dual luciferase luminescence measurement, 50 µl of a cell culture (5×10⁵ cells) were transferred into a 96-well plate and lysed through addition of 20 µl passive lysis buffer (Promega) for one minute, following the instruction of the supplier. FF luminescence readout was detected upon addition of 50 µl of LAR II substrate, and then REN luminescence signal was recorded after the addition of 50 µl Stop & Glo^{®} substrate. Raw data were recorded as CSV files and the data were further processed as described in the statistics section.

For the screen, two biological replicates per variant of ribosomal protein strain, each resulting from independent yeast transformations, were measured in replicates to deliver 12 luciferase signal read-outs for production levels of TE-FF and REN, respectively. Expression levels of TE-FF and REN in wild type cells were recorded in parallel.

### Statistical analysis

Luciferase read-out data were sorted in Excel format, and statistical analysis was performed using MiniTab 21 program suite, and Microsoft Excel. The individual reporter luciferase readouts from each of the variant ribosomal protein deletion strains were first subjected to outlier statistics using the Grubbs test, based on the hypothesis that data collected are part of a larger normal distribution. Descriptive statistics assessed mean, median, standard deviation, standard error of the mean, and quartiles of measured luciferase read-out values. Prospective variant ribosomal protein deletion strains which showed statistically significant increase in tropoelastin FF expression, while leaving REN levels unaltered, were subjected to interference statistics, employing ANOVA testing.

### Riboscreen^{®} assay as a tool to identify ribosomal protein targets for translational boost of tropoelastin.

In order to identify a ribosomal protein (RP) target that serves as small molecule target to boost protein production levels of tropoelastin the inventors used the Riboscreen^{®} technology Bauer JW, Brandl C, Haubenreisser O, Wimmer B, Weber M, Karl T, Klausegger A, Breitenbach M, Hintner H, von der Haar T, Tuite MF, Breitenbach-Koller L. Specialized yeast ribosomes: a customized tool for selective mRNA translation. PLoS One. 2013 Jul 8;8(7):e67609. doi: 10.1371/journal.pone.0067609. PMID: 23861776; PMCID: PMC3704640; EP2251437). As a first-line cellular readout vehicle, the Riboscreen^{®} technology uses the yeast Saccharomyces cerevisiae, a well-established, cost-efficient host for heterologous human protein expression (Mokdad-Gargouri R, Abdelmoula-Soussi S, Hadiji-Abbès N, Amor IY, Borchani-Chabchoub I, Gargouri A. Yeasts as a tool for heterologous gene expression. Methods Mol Biol. 2012;824:359-70. doi: 10.1007/978-1-61779-433-9_18. PMID: 22160908). This is combined with a dual luciferase assay, which includes two independently expressed reporter proteins. In the present invention, one reporter carries the tropoelastin sequence, C-terminally tagged with a firefly luciferase (FF) reporter (TE-FF), and a second reporter REN, which carries a renilla luciferase reporter is used as well. To screen for an altered ribosomal protein function triggering increased production levels of tropoelastin, the inventors tested ribosomal proteins in the heterozygous state to serve as a proxy for altered functional availability of ribosomal proteins. Therefore, a series of yeast strain vehicles derived from diploid strain BY4743 was used, each strain being heterozygous for one or the other of the eighty cytoplasmic ribosomal protein genes. This constituted the ribosomal protein variant strain (RVS) screening platform.

In yeast, two thirds of cytoplasmic ribosomal proteins are encoded by paralogous genes, and therefore the RVS collection used in this screen included the respective paralogues which were available and grew under the conditions used in the experiments. This delivered a screening tool of 129 RVS. Each RVS vehicle would therefore be expected to have a sub-population of 'specialized' ribosomes as a consequence of reduction in ribosomal protein (RP) gene dosage, in a background of unaltered ribosomes (Genuth NR, Barna M. The Discovery of Ribosome Heterogeneity and Its Implications for Gene Regulation and Organismal Life. Mol Cell. 2018 Aug 2;71(3):364-374. doi: 10.1016/j.molcel.2018.07.018. PMID: 30075139; PMCID: PMC6092941; Norris K, Hopes T, Aspden JL. Ribosome heterogeneity and specialization in development. Wiley Interdiscip Rev RNA. 2021 Jul;12(4):e1644. doi: 10.1002/wrna.1644. Epub 2021 Feb 9. PMID: 33565275; PMCID: PMC8647923). In this way, a relative subtle interference with overall ribosome function can be achieved, comparable to nature's modular activation of a RP by post-translational modifications. This approach excludes the use of haploid RP gene deletions, as these would generate ribosomes completely devoid of a given ribosomal protein, a condition that often is associated with lethality in yeast and human cells. The eighty eukaryotic cytoplasmic ribosomal proteins are highly conserved from yeast to man in sequence, structure and ribosomal topology (Natchiar SK, Myasnikov AG, Kratzat H, Hazemann I, Klaholz BP. Visualization of chemical modifications in the human 80S ribosome structure. Nature. 2017 Nov 23;551(7681):472-477. doi: 10.1038/nature24482. Epub 2017 Nov 15. PMID: 29143818) and are functionally interchangeable (Pollutri D, Penzo M. Ribosomal Protein L10: From Function to Dysfunction. Cells. 2020 Nov 19;9(11):2503. doi: 10.3390/cells9112503. PMID: 33227977; PMCID: PMC7699173). This makes ribosomal proteins an attractive pool of targets to be screened in cost effective yeast vehicles for modulating production levels of proteins of interest.

### Tropoelastin serves as suitable luciferase-based protein production reporter

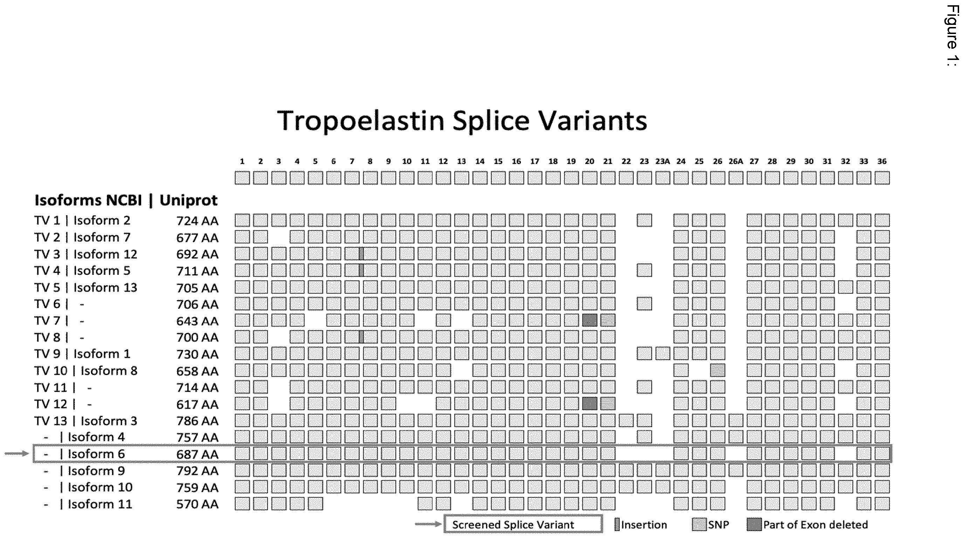

Tropoelastin (TE) the subunit of the elastin fiber, is a soluble 60-70 kDa protein and is encoded by the ELN gene, which in the human genome is located on the long arm of chromosome 7q11.2 (Ozsvar J, Yang C, Cain SA, Baldock C, Tarakanova A, Weiss AS. Tropoelastin and Elastin Assembly. Front Bioeng Biotechnol. 2021 Feb 25;9:643110. doi: 10.3389/fbioe.2021.643110. PMID: 33718344; PMCID: PMC7947355). Human ELN gives rise to a broad variety of splice isoforms that result in 13 known human isoforms of the mature tropoelastin protein. Indeed, variations in the relative abundance of alternatively spliced ELN mRNA transcripts and their corresponding protein isoforms have been documented between tissues, and this diversity is thought to be necessary for the fine tuning of elastogenesis in order to provide unique functional mechanical characteristics to different tissues (Reichheld SE, Muiznieks LD, Lu R, Sharpe S, Keeley FW. Sequence variants of human tropoelastin affecting assembly, structural characteristics and functional properties of polymeric elastin in health and disease. Matrix Biol. 2019 Nov;84:68-80. doi: 10.1016/j.matbio.2019.06.010. Epub 2019 Jun 26. PMID: 31254613). The formation of elastin fibers proceeds in distinct phases; tropoelastin synthesis, coacervation, cross-linking, and deposition. The extracellular cross-linking and deposition is guided by a suite of extracellular matrix enzymes and components, which all have to be present in the correct stoichiometry in order to avoid aggregation and breakdown products of unassembled elastin. While TE gene expression is highest during fetal and early neonatal development, low and steady state levels of tropoelastin expression and turnover are reported in adult tissues as well and also in cells isolated form adult tissues (Sproul EP, Argraves WS. A cytokine axis regulates elastin formation and degradation. Matrix Biol. 2013 Mar 11;32(2):86-94. doi: 10.1016/j.matbio.2012.11.004. Epub 2012 Nov 13. PMID: 23160093; PMCID: PMC3633528). In different diseases such as pulmonary hypertension, cigarette smoke induced emphysema or severe chronic obstructive pulmonary disease (COPD), tropoelastin synthesis is reactivated, although elastin production is disorganized and dysfunctional. In a similar fashion, photo aging induces unscheduled tropoelastin production, and specifically an isoform containing exon 26A, which is not upregulated upon treatment with retinoic acid, a topically applied skin rejuvenation regime, which increases production of tropoelastin isoforms lacking exon 26A and beneficially increases functional elastin (Rossetti D, Kielmanowicz MG, Vigodman S, Hu YP, Chen N, Nkengne A, Oddos T, Fischer D, Seiberg M, Lin CB. A novel anti-ageing mechanism for retinol: induction of dermal elastin synthesis and elastin fibre formation. Int J Cosmet Sci. 2011 Feb;33(1):62-9. doi: 10.1111/j.1468-2494.2010.00588.x. PMID: 20704601). However, prolonged therapy using retinoids, i.e., derivatives of vitamin A, is associated with irritant reactions such as burning, scaling or dermatitis limiting their acceptance by patients. These observations encouraged the present inventors to use tropoelastin isoform lacking exon 26A (Fig. 1), namely the commercially available isoform VI.

In order to systematically quantify protein expression levels of tropoelastin in each of the 129 RVS vehicles using Riboscreen^{®} technology, a luciferase reporter was generated using TE isoform VI C-terminally tagged with an FF luciferase tag (TE-FF). A second plasmid expressing the REN firefly reporter was used as an independently expressed control protein. This reporter pair is further engineered to harbor identical 5' and 3' regulatory untranslated regions (UTRs), derived from yeast *ADH1* mRNA and which harbor regulatory sequences mediating robust translation initiation and termination, respectively (Ruohonen L, Aalto MK, Keränen S. Modifications to the ADH1 promoter of Saccharomyces cerevisiae for efficient production of heterologous proteins. J Biotechnol. 1995 May 1;39(3):193-203. doi: 10.1016/0168-1656(95)00024-k. PMID: 7766401).

In order to monitor any possible changes in protein production levels in the RVS vehicles, the inventors first optimized and validated the luminescence readouts of the companion TE-FF and REN reporters in wild type cells in order to show functionality of the protein expression reporter pair. In wild type vehicles of a given experimental series and in a prototypical experiment with 6 replicates, the mean luminescence readout of TE-FF reporter protein expression when co-expressed in the presence of the companion REN reporter was 1.13*10^4 luciferase light unit counts and that of the REN reporter was 7.80*10^7 luciferase light unit counts. Such a difference in luciferase signal strength between REN and FF reporters, where the FF reporter is coupled to a large, heterologous human protein, has been reported from the inventors earlier prototypic screen, investigating the skin anchor protein Lamb3. Nevertheless, luciferase reporter protein expression levels of tropoelastin in wild type vehicles are thought to deliver faithful documentation of protein production levels of this heterologous human protein. The effect of a ribosomal protein modification on change in production levels of a protein of interest is reported to be in the range about 2-2.5-fold up and 2-2.5-fold down (Bauer JW, Brandl C, Haubenreisser O, Wimmer B, Weber M, Karl T, Klausegger A, Breitenbach M, Hintner H, von der Haar T, Tuite MF, Breitenbach-Koller L. Specialized yeast ribosomes: a customized tool for selective mRNA translation. PLoS One. 2013 Jul 8;8(7):e67609. doi: 10.1371/journal.pone.0067609. PMID: 23861776; PMCID: PMC3704640; Shi Z, Fujii K, Kovary KM, Genuth NR, Röst HL, Teruel MN, Barna M. Heterogeneous Ribosomes Preferentially Translate Distinct Subpools of mRNAs Genome-wide. Mol Cell. 2017 Jul 6;67(1):71-83.e7. doi: 10.1016/j.molcel.2017.05.021. Epub 2017 Jun 15. PMID: 28625553; PMCID: PMC5548184). Both FF luciferase and Ren luciferase signals without reporter protein are saturating at about 1* 10^9 luciferase light counts. Thus, luciferase assays screening for a 2-fold change in boost of production levels of TE_FF are well covered by a wild-type baseline protein production signal of TE-FF and REN as reported above.

### Riboscreen^{®} identified ribosomal protein rpL40 as candidate ribosomal target for boosting tropoelastin protein production levels.

Luminescence signal readouts of the FF-based tropoelastin reporter and the independently expressed REN reporter were recorded in the set of 129 RVS vehicles. Luciferase intensities were exported as CSV files and compiled in an excel overview sheet, providing in addition to raw data information on time of processing and OD600 values of cultures at time of harvest for measurement. Then, in a first step of statistical analysis, outlier analysis and descriptive statistical analysis of the data set (mean, median, standard deviation, standard error) was performed.

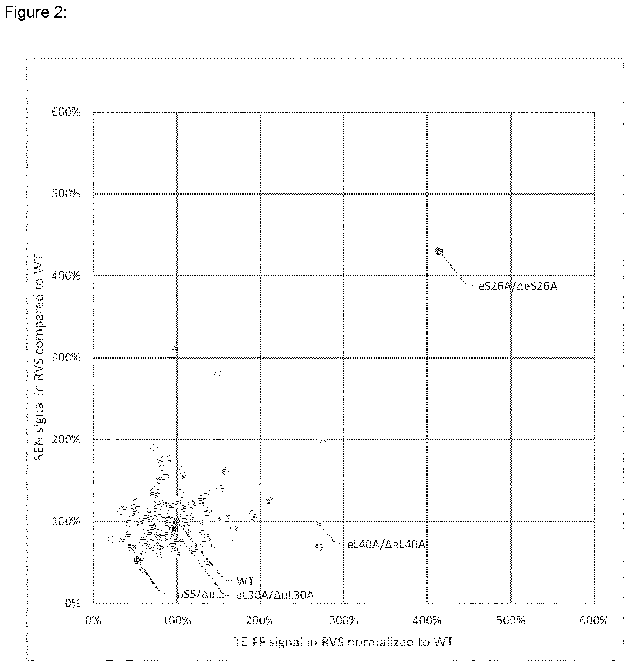

In order to reduce complexity of dual luciferase readout data obtained in the collection of RVS vehicles, a Pearson product moment (p) was generated to query the set of RVS variation of protein production levels of companion reporters TE-FF and REN. A ρ = 0.64 was calculated, which indicates for the majority of RVS a similar protein production level of TE-FF and REN reporters. While a ρ = 0.64 does not yet inform whether these similar production levels of the companion reporters are higher or lower than what is seen in wild type, these findings add weight to the situation that only a few RVS harbor ribosomes so specialized that altered availability of a given ribosomal protein triggers changes in protein production levels of either TE-FF or REN, respectively. To identify those ribosomal RVS that boost TE-FF production, but do not alter REN protein expression levels, a correlation plot was generated Accordingly, for each recorded RVS, mean TE-FF expression level normalized to WT expression level was plotted against the respective mean companion REN expression level normalized to WT. This captures for every RVS companion reporter protein production levels in a single data point in the correlation plot (Fig. 2). Fig 2 indicated that the majority of RVS, each one defined by a heterozygous depletion of a ribosomal protein, had little or no impact on a change in protein expression level of either reporter. Indeed, companion reporter expression signals obtained from RVS "inert" to appreciable change in protein production levels of either TE-FF or REN map close to the WT in a region defined by a circle of radius 0.5 fold difference in protein production level. This area of "inert" RVS harbors a majority of 104 RVS companion reporter protein expression signals, i.e., 80%, out of 129 RVS. Of the 26 RVS showing greater than 0.5-fold change in protein production levels in comparison to the WT, are RVS' that either decrease both reporters or increase both reporters (strain eS26A/ΔeS26A), and thus follow the p = 0.64 Pearson product moment. However, the inventors aimed to identify a VRS carrying a ribosomal protein depletion that selectively increases TE-FF protein expression levels, while leaving REN expression levels unaltered Again, Fig. 2 shows that such a candidate specialization of ribosomes is generated by depletion of ribosomal protein L40 as encoded by the RPL40A gene.

To provide a more robust statistical analysis, the data were subjected to t-test analysis, and ANOVA This confirmed the RVS RPL40A/delta RPL40A as a candidate vehicle to carry a gene dosage modification of rpL40, which in comparison to WT triggers a significant boost of 2.6 fold of signal strength and protein production levels of the TE-FF reporter, while leaving REN expression level unaltered. To visualize this observation, luciferase readout signals for companion TE-FF and REN reporters, respectively, are first shown individually for selected vehicles, WT, RVS RPL40A/delta RPL40A, and RVS RPL30/delta RPL30. To arrive at a standardized comparison, TT-FF signals and REN signals were normalized to their respective reporter signals in the wild type (Fig. 3).

To arrive at a fold level presentation of tropoelastin production levels in selected vehicles, the ratio of TE-FF/REN protein production level was calculated and normalized to wild type. This showed a significant 2.6-fold increase for TE-FF protein production levels in the RVS depleted for rpL40 in comparison to wild type (Fig. 3). The inventors conclude that out of the 129 RVS tested, in fact only one candidate RVS, with altered functional availability of rpL40, selectively boosts production levels of tropoelastin 2.6-fold.

### Ribosomal protein L40 as a candidate drug target for boosting TE levels

Similar to all of the 80 eukaryotic cytoplasmic ribosomal proteins, ribosomal protein L40 is highly conserved in sequence and structure between yeast and humans. Indeed, comparative sequence alignment of the ribosomal proteins encoded by the yeast paralogous genes RPL40A and RPL40B and the human single copy gene hRPL40 demonstrates high sequence identity both between the yeast paralogous proteins and the human ortholog. The Rpl40 ribosomal protein, both in yeast and in human ribosomes, binds to a highly conserved part of 25S/28S ribosomal RNA (rRNA) of the large ribosomal subunit called the sarcin ricin loop, which is the docking site for translation elongation factors. On the assembled, translation competent ribosome, the Rpl40 protein occupies the identical topological position, i.e., the docking site for elongation factors. Visual comparative inspection of rpL40 on the yeast and human ribosome shows that this protein resides at the entrance of the intersubunit tunnel, where during protein synthesis the elongation factors load the incoming tRNAs into the ribosome. The yeast rpL40 is positioned slightly closer to the bottom rim of the ribosome. This is because in the human ribosome the human rRNA expansion segments must arrange in a way that preserves the relative position of the ribosomal function sites, including the sarcin ricin loop domain, the docking site of the elongation factors. The inventors conclude that human rPL40, identified with Riboscreen^{®} technology as a target ribosomal protein to boost production levels of tropoelastin, serves as a target for small molecules to customize protein expression levels, in particular of the precursor of elastin.

### Molecules targeting ribosomal protein rpL40 as identified

The compounds that were identified in the context of the present invention using a screening library of small molecules were as follows.

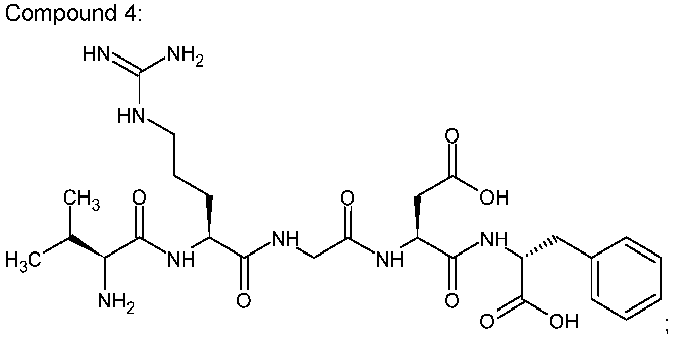

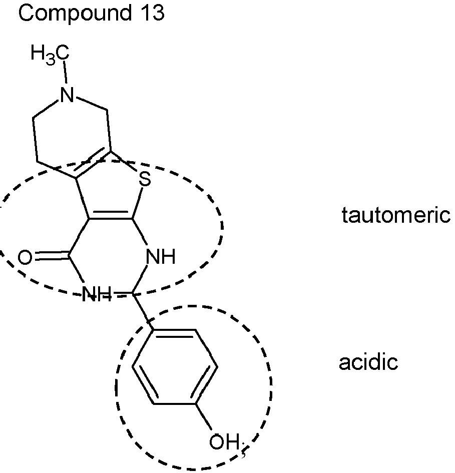

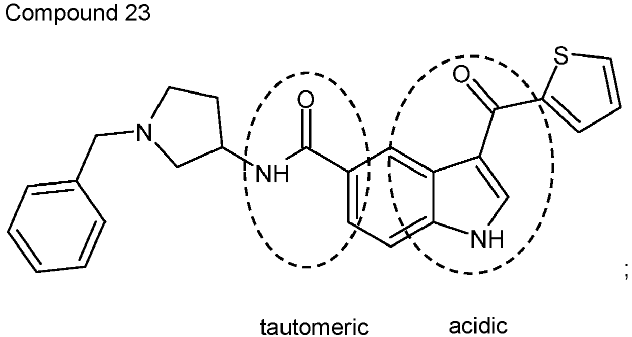

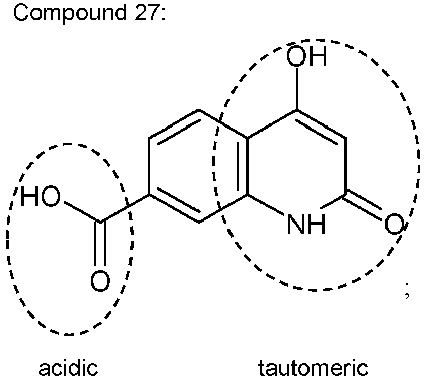

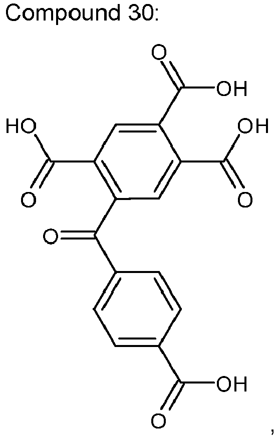

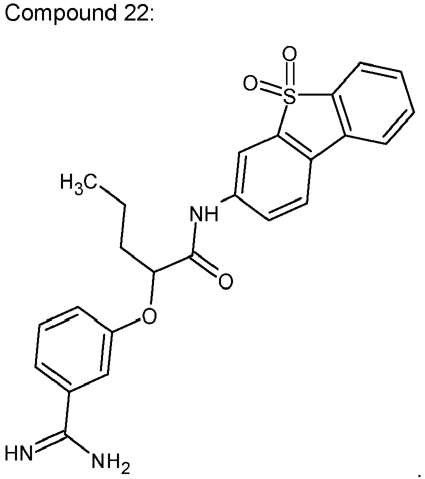

In the above formulae, the dashed circles indicate a group (HN-CO-C=C-OH) [that is tautomeric within about 3-4Å distance to an acidic group in the same molecular plane. This molecular similarity was identified for compounds 13, 23, 27, and also 4 (for some configurations). Compounds 30 and 22 are chemically different.

## Claims

1. A method for identifying a cosmetically and/or pharmaceutically active compound that modulates the rpL40-dependent translation of at least one mRNA encoding for a tropoelastin in a mammalian cell, comprising
a) contacting rpL40 or a functional fragment thereof with at least one candidate compound in the presence of said at least one mRNA to be translated, and
b) detecting the modulation of the translation of said at least one mRNA encoding for a tropoelastin compared to the translation in the absence of said at least one candidate compound, wherein a modulation of the translation of said at least one mRNA is indicative for a cosmetically and/or pharmaceutically active compound.

2. The method according to claim 1, furthermore comprising the step of a pre-identifying the translation of said at least one mRNA as being rpL40-dependent.

3. The method according to claim 1 or 2, wherein said modulation leads to an increase or decrease of said rpL40-dependent translation of said at least one mRNA.

4. The method according to any one of claims 1 to 3, furthermore comprising detecting a binding of said at least one candidate compound to rpL40, preferably to an isolated or partially isolated rpL40, or to rpL40 in the context of the ribosomal subunit or in the context of both subunits of the mammalian ribosome.

5. The method according to any one of claims 1 to 4, furthermore comprising detecting a binding of said at least one candidate compound to a fragment of rpL40, wherein said fragment comprises from about 30 to 90, preferably 60 to 80, most preferred about 50 of the C-terminal amino acids of the mammalian rpL40 protein, preferably according to SEQ ID NO: 2.

6. The method according to any one of claims 1 to 5, wherein said detecting of binding to rpL40 or the fragment thereof is performed as a pre-screening before contacting said at least one candidate compound with said rpL40.

7. The method according to any one of claims 1 to 6, furthermore comprising a pre-selection step comprising molecular modeling of said binding of said at least one candidate compound to rpL40 or a fragment thereof, for example using a computer program, such as SwissDock.

8. The method according to any one of claims 1 to 7, wherein said rpL40 or fragment thereof is human rpL40.

9. The method according to any one of claims 1 to 8, wherein said method is performed in vitro, in cell culture or in vivo, preferably in a non-human mammal.

10. The method according to any one of claims 1 to 9, wherein said candidate compound is selected from a chemical substance, a substance selected from a peptide library, a library of small organic molecules, a combinatory library, a cell extract, in particular a plant cell extract, a "small molecular drug", a natural compound, a protein and/or a protein fragment, and an antibody or fragment thereof, and in particular from the group of compounds selected from the formulae: 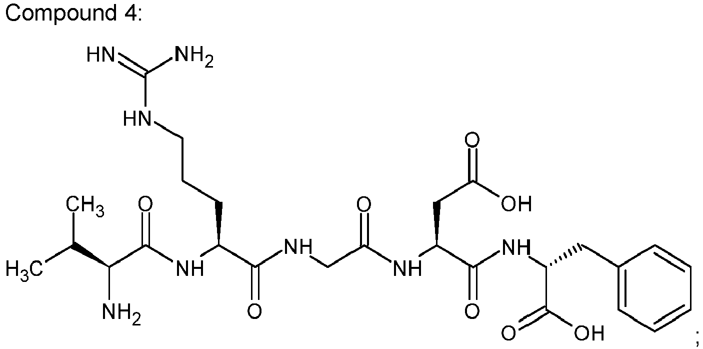 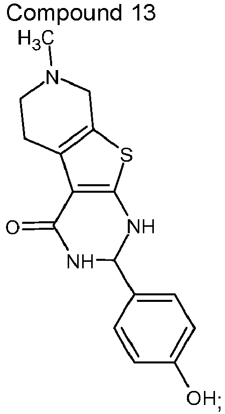 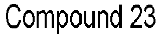 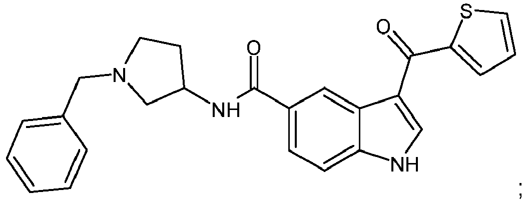 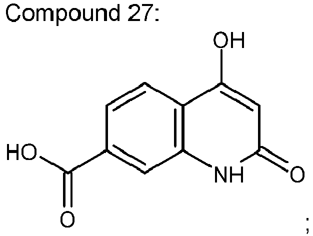 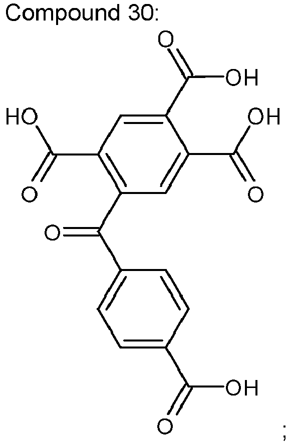 and 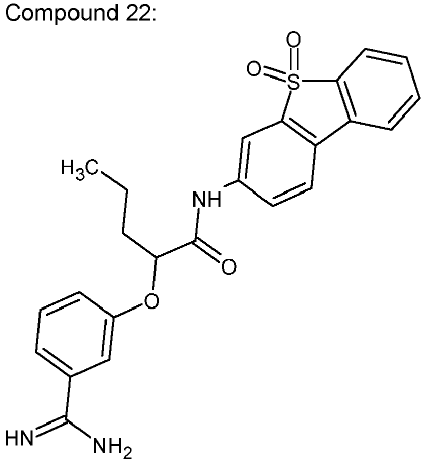 and derivatives thereof, and cosmetically and/or pharmaceutically acceptable salts thereof

11. The method according to any one of claims 1 to 10, wherein said at least one mRNA encodes for a tropoelastin isoform, in particular tropoelastin isoform VI.

12. A screening system for identifying a cosmetically and/or pharmaceutically active compound that modulates the rpL40-dependent translation of at least one mRNA encoding for a tropoelastin in a mammalian cell, comprising
an eukaryotic cell recombinantly expressing a mammalian rpL40 or a fragment of a mammalian rpL40, wherein said fragment comprises from about 30 to 90, preferably 60 to 80 of the C-terminal amino acids of the mammalian rpL40 protein, preferably according to SEQ ID NO: 2,
an expression construct for recombinantly expressing the at least one mRNA to be tested, and
optionally, one or more candidate compounds to be tested.

13. The screening system according to claim 12, wherein said eukaryotic cell is selected from a yeast, insect, rodent, or human cell, in particular a keratinocyte.

14. The screening system according to claim 12 or 13, wherein said eukaryotic cell is an inactivation or depletion mutant of rpL40.

15. A compound that modulates the rpL40-dependent translation of at least one mRNA encoding for a tropoelastin in a mammalian cell for use in the prevention or treatment of diseases, in particular a disease or condition caused by a lack and/or insufficient synthesis of functional elastin, wherein said disease or condition is preferably selected from pulmonary hypertension, cigarette smoke induced emphysema, undesirable would healing, and severe chronic obstructive pulmonary disease (COPD), and wherein said compound was identified with a method according to any one of claims 1 to 11, and in particular from the group of compounds selected from the following formulae: 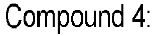 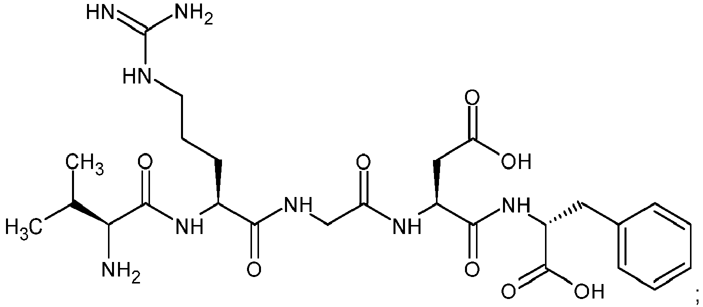 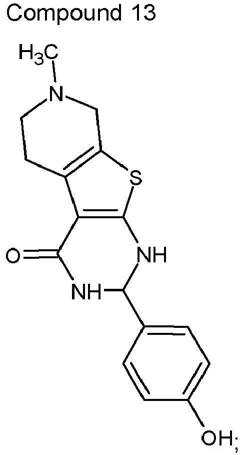 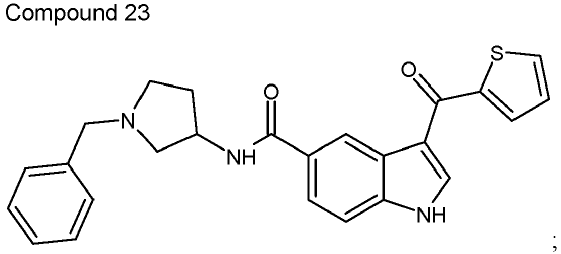 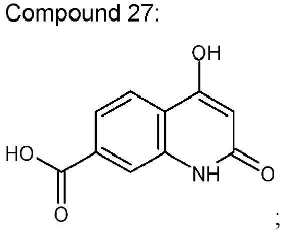 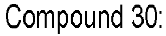 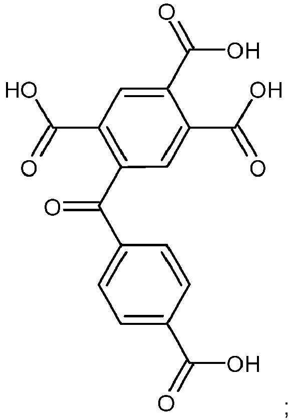 and 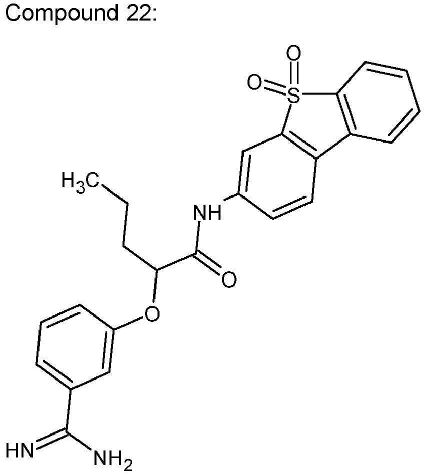 and derivatives thereof, and cosmetically and/or pharmaceutically acceptable salts thereof

16. A method, in particular a cosmetic or non-medical method, for modulating the rpL40-dependent translation of at least one mRNA encoding for a tropoelastin in a mammalian cell or tissue, comprising contacting said cell or tissue with an effective amount of a compound that was identified with a method according to any one of claims 1 to 11, or the cosmetic and/or pharmaceutical composition according to claim 16.

17. The method according to claim 16, wherein said modulating the rpL40-dependent translation of at least one mRNA encoding for a tropoelastin is for preventing or treating a condition selected from photo ageing, skin rejuvenation, and increasing functional elastin synthesis.

18. A method of preventing, treating or ameliorating a disease or condition caused by a lack and/or insufficient synthesis of functional elastin, wherein said disease or condition is preferably selected from pulmonary hypertension, cigarette smoke induced emphysema, and severe chronic obstructive pulmonary disease (COPD), comprising administering an effective amount of a compound that was identified with a method according to any one of claims 1 to 11, to a patient or subject in need of said treatment or prevention.
